(19) Europäisches Patentamt
European Patent Office
Office européen des brevets

(11) EP 3 078 979 B1

(12) **EUROPÄISCHE PATENTSCHRIFT**

(45) Veröffentlichungstag und Bekanntmachung des Hinweises auf die Patenterteilung:
**20.09.2023 Patentblatt 2023/38**

(21) Anmeldenummer: **16158221.8**

(22) Anmeldetag: **02.03.2016**

(51) Internationale Patentklassifikation (IPC):
**G01R 33/563** (2006.01) **G01R 33/565** (2006.01)

(52) Gemeinsame Patentklassifikation (CPC):
**G01R 33/56509; G01R 33/56341**

(54) **GESCHWINDIGKEITSKOMPENSIERTE MR-DIFFUSIONSBILDGEBUNG**

VELOCITY-COMPENSATED DIFFUSION MR IMAGING

IMAGERIE DE DIFFUSION PAR RM AVEC COMPENSATION DE VITESSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.03.2015 DE 102015205693**

(43) Veröffentlichungstag der Anmeldung:
**12.10.2016 Patentblatt 2016/41**

(73) Patentinhaber: **Siemens Healthcare GmbH 91052 Erlangen (DE)**

(72) Erfinder: **Stemmer, Alto 91054 Erlangen (DE)**

(56) Entgegenhaltungen:
WO-A1-2013/025487    DE-A1-102012 217 992
US-A1- 2008 275 329

• ERIC ALIOTTA ET AL: "Convex optimized diffusion encoding (CODE) gradient waveforms for minimum echo time and bulk motion-compensated diffusion-weighted MRI", MAGNETIC RESONANCE IN MEDICINE., 1. Februar 2016 (2016-02-01), Seiten n/a-n/a, XP055331381, US ISSN: 0740-3194, DOI: 10.1002/mrm.26166

**Beschreibung**

Die Erfindung betrifft ein Verfahren zur Ansteuerung eines Magnetresonanzbildgebungssystems zur Erzeugung von Magnetresonanzbilddaten eines Untersuchungsobjekts, bei dem Magnetresonanzrohdaten erfasst werden. Schließlich betrifft die Erfindung ein Magnetresonanzbildgebungssystem.

Bei der Erzeugung von Magnetresonanzaufnahmen wird der zu untersuchende Körper einem relativ hohen Grundmagnetfeld, beispielsweise von 1,5 Tesla, 3 Tesla, oder bei neueren Hochmagnetfeldanlagen sogar von 7 Tesla und höher, ausgesetzt. Es wird dann mit einer geeigneten Antenneneinrichtung ein hochfrequentes Anregungssignal ausgesendet, welches dazu führt, dass die Kernspins bestimmter durch dieses Hochfrequenzfeld in dem gegebenen Magnetfeld resonant angeregter Atome um einen bestimmten Flipwinkel gegenüber den Magnetfeldlinien des Grundmagnetfelds verkippt werden. Das bei der Relaxation der Kernspins abgestrahlte Hochfrequenzsignal, das sog. Magnetresonanzsignal, wird dann mit geeigneten Antenneneinrichtungen, welche auch identisch mit der Sendeantenneneinrichtung sein können, aufgefangen. Die so akquirierten Rohdaten werden nach Demodulation und Digitalisierung schließlich genutzt, um die gewünschten Bilddaten zu rekonstruieren. Zur Ortskodierung werden dem Grundmagnetfeld während des Sendens und des Auslesens bzw. Empfangens der Hochfrequenzsignale jeweils definierte Magnetfeldgradienten überlagert.

Üblicherweise setzt sich eine Magnetresonanzaufnahme aus einer Vielzahl von einzelnen Teilmessungen zusammen, bei denen Rohdaten aus verschiedenen Schichten des Untersuchungsobjekts aufgenommen werden, um daraus anschließend Volumenbilddaten zu rekonstruieren.

Darüber hinaus ist es aber bei vielen Untersuchungen auch notwendig, mehrere, d. h. eine ganze Serie von Magnetresonanzaufnahmen des Untersuchungsobjekts, durchzuführen, wobei ein bestimmter Messparameter variiert wird. Anhand der Messungen wird die Wirkung dieses Messparameters auf das Untersuchungsobjekt beobachtet, um daraus dann später diagnostische Schlüsse zu ziehen. Unter einer Serie sind dabei zumindest zwei, in der Regel aber mehr als zwei Magnetresonanzaufnahmen zu verstehen. Sinnvollerweise wird dabei ein Messparameter so variiert, dass der Kontrast eines bei den Messungen angeregten bestimmten Materialtyps, beispielsweise eines Gewebetyps des Untersuchungsobjekts oder eines chemischen Stoffes, der signifikant für die meisten bzw. bestimmte Gewebetypen ist, wie z. B. Wasser, durch die Variation des Messparameters möglichst stark beeinflusst wird. Dies sorgt dafür, dass die Wirkung des Messparameters auf das Untersuchungsobjekt besonders gut sichtbar ist.

Ein typisches Beispiel für Serien von Magnetresonanzaufnahmen unter der Variation eines den Kontrast stark beeinflussenden Messparameters sind so genannte Diffusionsbildgebungsverfahren (englisch "Diffusion weighting imaging" (DWI)). Unter Diffusion versteht man die Brownsche Bewegung (englisch "brownian motion") von Molekülen in einem Medium. Bei der Diffusionsbildgebung werden in der Regel mehrere Bilder mit unterschiedlichen Diffusionsrichtungen und -gewichtungen aufgenommen und miteinander kombiniert. Die Stärke der Diffusionswichtung wird meist durch den so genannten "b-Wert" definiert. Die Diffusionsbilder mit unterschiedlichen Diffusionsrichtungen und -gewichtungen bzw. die daraus kombinierten Bilder können dann zu diagnostischen Zwecken verwendet werden. So können durch geeignete Kombinationen der aufgenommenen diffusionsgewichteten Bilder Parameterkarten mit besonderer diagnostischer Aussagekraft erzeugt werden, wie beispielsweise Karten, die den "Apparent Diffusion Coefficient (ADC)" oder die "Fractional Anisotropy (FA)" wiedergeben.

Häufig basiert die Diffussionsbildgebung auf der Echoplanarbildgebung (EPI) wegen der kurzen Akquisitionszeit der EPI Sequenz pro Bild und Ihrer Robustheit gegenüber Bewegung. Bei einer Diffusionsbildgebung mit EPI findet man auch ohne Bewegung des Patienten, die zusätzlich eine Rolle spielen kann, in den diffusionsgewichteten Bildern aufgrund von lokalen $B_0$-Inhomogenitäten und residuellen Wirbelstromfeldern Verzerrungen. Letztere hängen von der Richtung und der Stärke der Diffusionswichtung ab. Diese Verzerrungen können zu Fehlern in den ausgewerteten Diffusionskarten führen.

Bei der diffusionsgewichteten Bildgebung werden zusätzliche Gradienten in eine Pulssequenz einfügt, um die Diffusionseigenschaften des Gewebes sichtbar zu machen oder zu messen. Diese Gradienten führen dazu, dass Gewebe mit schneller Diffusion (z.B. Zerebrospinalflüssigkeit, englisch "cerebrospinal fluid" CSF) einem stärkeren Signalverlust unterliegt als Gewebe mit langsamer Diffusion (z.B. die graue Substanz im Gehirn, englisch "grey matter"). Der daraus resultierende Diffusionskontrast wird klinisch immer bedeutender und Anwendungen gehen inzwischen weit über die klassische frühe Erkennung von ischämischem Schlaganfall (englisch "ischemic stroke") hinaus.

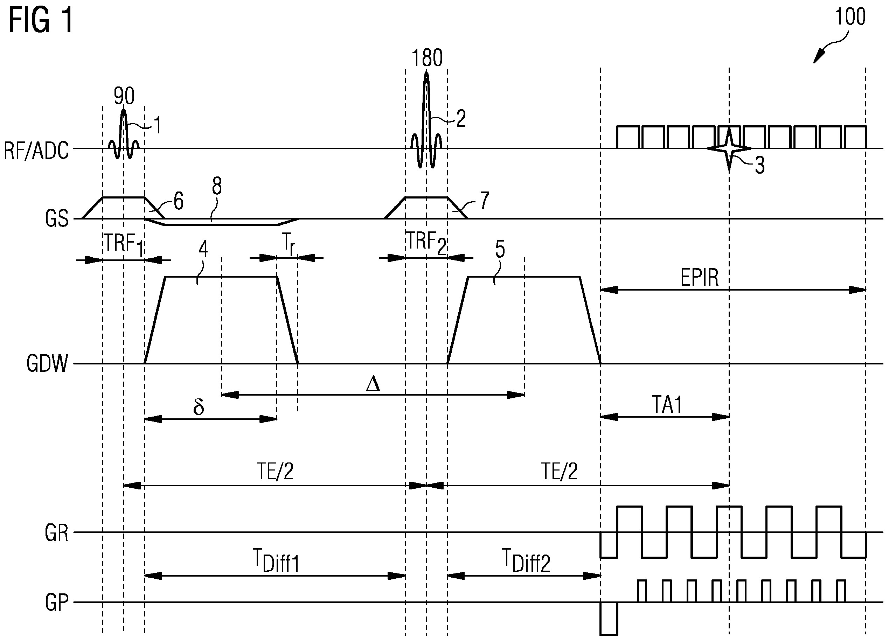

Eine typische Pulssequenz für die Diffusionsbildgebung ist die Stejskal-Tanner-Diffusionssequenz, die in Figur 1 dargestellt ist. Allerdings kommt es bei der Anwendung einer solchen Pulssequenz bei der diffusionsgewichteten Bildgebung der Leber und des Herzens zu Signalverlusten, welche sich auf die Herzbewegung zurückführen lassen. Diese Signalverluste nehmen mit der Diffusionswichtung zu und führen damit zu einer Überschätzung des scheinbaren Diffusionskoeffizienten (ADC - englisch "apparent diffusion coefficient"). Das ist insbesondere dann ein Problem, wenn der ADC-Wert als Diskriminator zwischen harmlosen und bösartigen Läsionen verwendet wird.

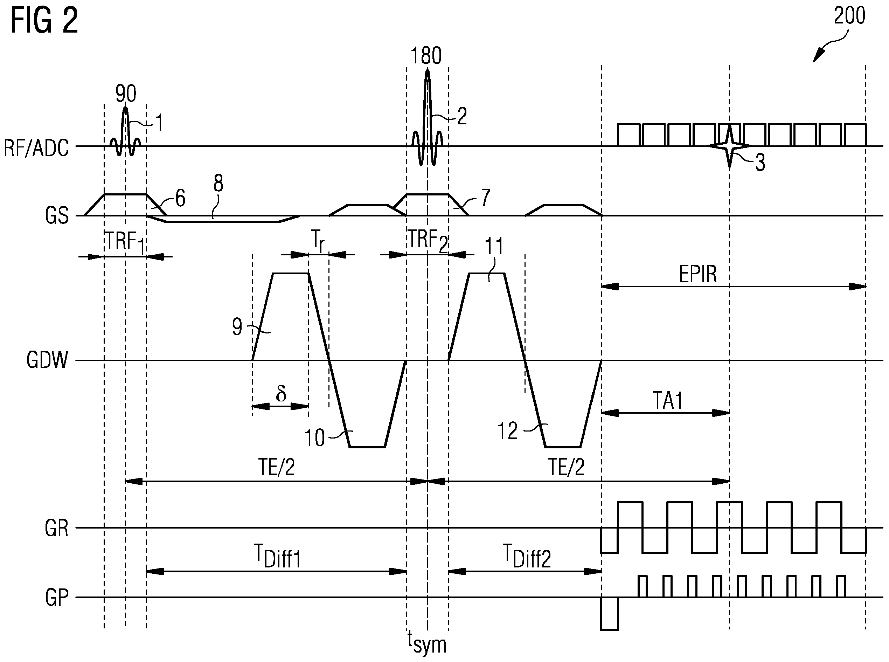

Eine Möglichkeit, die Artefakte in Folge der Herzbewegung (und anderer makroskopischer Bewegungen) signifikant zu reduzieren, besteht darin, die Standard-Stejskal-Tanner-Diffusionssequenz mit unipolaren Gradienten durch eine Sequenz mit geschwindigkeitskompensierten (englisch "velocity compensated") bipolaren Gradienten zu ersetzen, wie sie in Figur 2 gezeigt ist. Der Hauptnachteil dieser geschwindigkeitskompensierten Sequenz besteht darin, dass das bipolare Gradientenschema dieser Sequenz eine signifikant geringere Diffusionssensitivität besitzt als das Stejskal-Tanner Schema. Daher muss zum Erreichen einer gewünschten Diffusionssensitivität die Gradientendauer um mindestens einen Faktor 1.6 verlängert werden. Dies führt zu einer Verlängerung der Echozeit der Sequenz und damit wegen des inhärenten T2-Zerfalls des Gewebes zu einer Verschlechterung des Signal/Rausch-Verhältnisses SNR, die nur über eine signifikant längere Messzeit kompensiert werden kann.

Eine Beschreibung der geschwindigkeitskompensierten Diffusionssequenz im Stand der Technik und ihrer Unempfindlichkeit gegenüber makroskopischer Bewegung ist in einem Konferenzbeitrag von C. Thomsen, P. Ring und O. Henriksen mit dem Titel "In vivo measurement of water self diffusion by magnetic resonance imaging", veröffentlicht in "Proceedings of the Seventh Scientific Meeting, Society of Magnetic Resonance in Medicine," Seite 890, San Francisco, CA (1988), zu finden. Die US20080275329A1 beschreibt ein weiteres Verfahren zur Diffusions-Präparation mit verschwindenden ersten Momenten.

Diese Sequenz wurde später in zahlreichen Veröffentlichungen eingesetzt, um Artefakte in Folge von makroskopischer Bewegung zu verringern. Eine aktuelle Veröffentlichung, in der die Sequenz in der diffusionsgewichteten Bildgebung der Leber eingesetzt wird, um Artefakte in Folge der Herzbewegung zu verringern, ist der Artikel von Masanori Ozaki et al.: "Motion Artifact Reduction of Diffusion-Weighted MRI of the Liver: Use of Velocity-Compensated Diffusion Gradients Combined With Tetrahedral Gradients", erschienen in der Zeitschrift JOURNAL OF MAGNETIC RESONANCE IMAGING, Heft 37, Seiten 172-178 (2013), DOI 10.1002/jmri.23796. Die Autoren diskutieren auch das oben genannte Problem, dass diese Sequenz für einen gewünschten b-Wert im Vergleich zu einer Stejskal-Tanner Sequenz eine um den Faktor 1,6 verlängerte Gradientenschaltung benötigt und somit, in Folge der verlängerten Echozeit, das SNR der Bilder reduziert ist. Die Autoren adressieren dieses Problem, indem sie eine richtungsunabhängige Diffusionspräparation einsetzen, bei der Gradienten auf allen drei Achsen gleichzeitig geschaltet werden. Sie erreichen damit eine TE-Verkürzung (und damit einen SNR-Gewinn) gegenüber einem richtungsunabhängigen Präparationsschema, bei dem jeweils nur Gradienten auf einer Achse geschaltet werden.

Weitere diffusionsgewichtete MR-Pulssequenzen sind aus US 2008/0275329 A1 bekannt.

Es sei erwähnt, dass eine stimulierte Echo-Präparation (STEAM von englisch "stimulated echo acquisition mode"), die eine gewünschte Diffusions-Sensibilisierung mit einer besonders kurzen Echozeit realisieren kann, nicht geschwindigkeitskompensiert ist und damit empfindlich gegenüber makroskopischer Bewegung ist. Es sei weiter erwähnt, dass unterschiedlich lange Zeiten zur Diffusions-Sensibilisierung vor und nach dem HF-Refokussierungspuls nicht die Ausnahme, sondern die Regel sind. Eine Symmetrisierung der beiden Zeiten (z.B. durch das Nichtakquirieren der frühen Zeilen eines EPI Auslesezugs) kann mit erheblichen Problemen verbunden sein, bis hin zu einem kompletten Signalverlust, falls das Echo im k-Raum in Folge von makroskopischer Bewegung während der Diffusions-Sensibilisierung in den nicht akquirierten Bereich verschoben wird.

Die Aufgabe der vorliegenden Erfindung besteht darin, ein Magnetresonanzbildgebungssystem zur Erzeugung von Magnetresonanzbilddaten eines Untersuchungsobjekts mit einer geschwindigkeitskompensierten Diffusionssequenz anzusteuern, mit der in gegebener Zeit eine größere Diffusionssensitivität realisierbar ist als bei herkömmlichen geschwindigkeitskompensierten Sequenzen oder alternativ eine gewünschte Diffusionssensitivität in kürzerer Zeit realisierbar ist.

Diese Aufgabe wird durch ein Verfahren gemäß Patentanspruch 1, durch ein Magnetresonanzbildgebungssystem gemäß Patentanspruch 12, sowie durch ein Computerprogrammprodukt gemäß Patentanspruch 13 und ein computerlesbares Medium gemäß Patentanspruch 14 gelöst.

Das erfindungsgemäße Verfahren zur Ansteuerung eines Magnetresonanzbildgebungssystems zur Erzeugung von Magnetresonanzbilddaten eines Untersuchungsobjekts, bei dem Magnetresonanzrohdaten erfasst werden, weist mindestens eine Diffusionskontrast-Pulssequenz auf. Weiterhin weist das erfindungsgemäße Verfahren mindestens einen Anregungsvorgang auf, bei dem ein Anregungs-HF-Puls erzeugt wird. Zudem weist das erfindungsgemäße Verfahren einen Auslesevorgang zur Akquisition von Magnetresonanzrohdaten auf, bei dem HF-Signale erfasst werden. Zusätzlich umfasst das erfindungsgemäße Verfahren einen Schritt zur Diffusions-Sensibilisierung unter Verwendung einer Diffusionskontrast-Gradientenpulssequenz mit einer ungeraden Anzahl $2n+1$ von Diffusionskontrast-Gradientenpulsen, wobei $n$ eine natürliche Zahl ist. Die ungerade Anzahl $2n+1$ von Diffusionskontrast-Gradientenpulsen werden dabei zeitlich sequentiell derart erzeugt, dass die Summe der nullten Gradientenmomente der Diffusionskontrast-Gradientenpulse den Wert Null aufweist und die Summe der ersten Gradientenmomente der Diffusionskontrast-Gradientenpulse den Wert Null aufweist. Außerdem wird zwischen zwei der Diffusionskontrast-Gradientenpulse genau ein HF-Refokussierungspuls geschaltet.

Unter dem n-ten Moment $m_n(t)$ einer Gradientenanordnung $G_i$ versteht man das Integral

$$m_n(t) = \int_0^t G_i(\tau) \cdot \tau^n d\tau \ . \qquad (1)$$

Damit erhält man für die Phase einer kleinen Probe, die sich zum Zeitpunkt 0 (Mitte des Anregungspulses) am Ort rc befindet und sich mit konstanter Geschwindigkeit $v_0$ durch das Messvolumen bewegt:

$$\phi(t) = 2\pi\gamma \int_0^t \vec{G}(\tau)\vec{r}(\tau)d\tau = 2\pi\gamma \int_0^t \vec{G}(\tau)[\vec{r}_0 + \vec{v}_o(\tau)]d\tau = 2\pi\gamma[\vec{m}_0\vec{r}_0 + \vec{m}_1\vec{v}_0] \qquad (2)$$

Bewegte Spins akquirieren also eine zusätzliche Phase φ die proportional zu ihrer Geschwindigkeit $v_0$ und zum ersten Moment der Gradientenanordnung G ist. Ist die Geschwindigkeit der verschiedenen Spins, die zu dem Signal eines Voxels bzw. Bildpunkts (Bildpunkt kann Pixel oder Voxel sein) beitragen, unterschiedlich, kann diese zusätzliche Phase φ zu einer Dephasierung des Signals und damit zu Auslöschungen in den berechneten Bildern führen.

Indem bei dem erfindungsgemäßen Verfahren das nullte und das erste Gradientenmoment $m_0(t)$, $m_1(t)$ der Diffusionskontrast-Gradientenpulssequenz jeweils den Wert Null aufweisen, wird die beschriebene Dephasierung des Signals vermieden.

Unter Diffusionskontrast-Gradienten sollen die bereits erwähnten, zusätzlich zu den für die Bildgeneration geschalteten Gradienten in eine Pulssequenz eingefügten Gradienten verstanden werden, die dazu dienen, die Diffusionseigenschaften des Gewebes sichtbar zu machen oder zu messen. Diese Diffusionskontrast-Gradienten werden im Folgenden oft einfach als "Gradienten" oder auch "Diffusionsgradienten" bezeichnet.

Mit der Wahl einer ungeraden Anzahl von Diffusionskontrast-Gradientenpulsen wird erreicht, dass die Summe der nullten Gradientenmomente zumindest eines Teils benachbarter Gradientenpulse in dem Zeitraum zwischen Anregungs-HF-Puls und HF-Refokussierungspuls und/oder in dem Zeitraum zwischen dem Zeitpunkt des HF-Refokussierungspulses und dem Zeitpunkt des Beginns des Auslesevorgangs nicht den Wert Null ergibt.

Dieser Sachverhalt wird genutzt, um Zeiträume zwischen den genannten benachbarten Gradientenpulsen zur Diffusionssensibilisierung zu nutzen und so zusätzlich zur Geschwindigkeitskompensation auch eine verbesserte Diffusionssensibilisierung zu erreichen.

Ziel der Diffusionswichtung ist es, das Signal von Gewebe mit schneller Diffusion (z.B. ungebundenes Wasser) stärker zu schwächen als Gewebe mit langsamer Diffusion. Wie eingangs erwähnt, ist die Diffusion in Läsionen häufig eingeschränkt. In den berechneten Bildern erscheinen diese Areale mit eingeschränkter Diffusion dann hell. Im Allgemeinen ist die Einschränkung der Diffusion im Gewebe richtungsabhängig. Mathematisch beschreibt man die Richtungsabhängigkeit der Diffusionseinschränkung des Gewebes als Tensor, den symmetrischen Diffusionstensor D:

$$D = \begin{bmatrix} D_{xx} & D_{xy} & D_{xz} \\ D_{xy} & D_{yy} & D_{yz} \\ D_{xz} & D_{yz} & D_{zz} \end{bmatrix} . \qquad (3)$$

Die Sensitivität des MR-Messverfahrens gegenüber der Diffusion ist durch die Richtung der Diffusionsgradienten ebenso richtungsabhängig und wird über einen weiteren Tensor b beschrieben:

$$b = \begin{bmatrix} b_{xx} & b_{xy} & b_{xz} \\ b_{xy} & b_{yy} & b_{yz} \\ b_{xz} & b_{yz} & b_{zz} \end{bmatrix} . \qquad (4)$$

Dabei sind die Komponenten des symmetrischen b-Tensors durch die k-Raum-Trajektorie der Sequenz bestimmt durch

$$b_{ij}(TE) = \int_0^{TE} k_i(\tau)k_j(\tau)d\tau \qquad (5)$$

mit

$$k_i(t) = 2\pi \int_0^t G_i(\tau)d\tau \; . \qquad (6)$$

Dabei ist $G_i(t)$ die Amplitude des in die Richtung i geschalteten Gradienten zum Zeitpunkt t und $\gamma$ das gyromagnetische Verhältnis, das für Protonen 42.576 MHz/T beträgt. Der Integrationsbereich verläuft von der Mitte des HF-Anregungspulses einer Pulssequenz bis zum Echozeitpunkt TE. Tatsächlich tragen alle geschalteten Gradienten zur Diffusionssensitivität der MR-Sequenz bei. Häufig schließt man in der Berechnung nur die explizit zur Diffusionswichtung geschalteten Gradienten, d.h. die Diffusionskontrast-Gradienten ein, da diese in der Regel höhere Amplituden und längere Dauer als die für die Bildgeneration geschalteten Gradienten haben und deshalb die Diffusionssensitivität dominieren.

Die Schwächung eines MR-Signals lässt sich damit wie folgt schreiben:

$$S = S_0 e^{-bD} \; . \qquad (7)$$

Dabei ist $S_0$ das Signal ohne Diffusion und bD das Tensorprodukt aus Diffussionstensor D des Gewebes und b-Tensor der Sequenz:

$$bD = \sum_{i=\{x,y,z\}} \sum_{j=\{x,y,z\}} b_{ij}D_{ij} \qquad (8)$$

Häufig schaltet man den Diffusionsgradienten entlang einer einzigen Achse oder man führt drei Messungen nacheinander durch, bei denen man den Diffusionsgradienten jeweils entlang einer von drei zueinander orthogonalen Richtungen schaltet. Schaltet man die Diffusionsgradienten dabei in dem Koordinatensystem, in dem auch der Diffusionstensor definiert ist, so erhält man aus Formel (7):

$$S_x = S_0 e^{-b_{xx}D_{xx}}$$
$$S_y = S_0 e^{-b_{yy}D_{yy}} \qquad (9)$$
$$S_z = S_0 e^{-b_{zz}D_{zz}}$$

Es trägt also jeweils nur ein einziges Element des b-Tensors zum MR-Signal bei. In diesem Zusammenhang nennt man dieses Element auch b-Wert $b = b_{xx} = b_{yy} = b_{zz}$ der Diffusionssequenz. Diesen b-Wert (sowie die anderen Elemente des Diffusionstensors) kann man mit Hilfe der Formeln (7) und (8) berechnen.

Anhand der Formeln (5) und (6) ist zu erkennen, dass ein Zeitabschnitt der Pulssequenz, bei dem kein Gradient geschaltet wird, zur Diffusionssensitivität nur dann beitragen kann, wenn das nullte Moment der Gradientenpulsfolge bis zu dem Beginn des jeweiligen Zeitabschnitts nicht den Wert Null aufweist.

Die Erfindung nutzt die Beobachtung, dass in der auf der Spin-Echo Technik basierten Diffusions-Bildgebung vor dem HF-Refokussierungspuls häufig mehr Zeit zur Diffusionspräparation zur Verfügung steht als nach dem HF-Refokussierungspuls. Die Gründe hierfür hängen von dem verwendeten Auslesemodul ab. Das in der klinischen Bildgebung bei Weitem wichtigste Auslesemodul ist ein Single-Shot-Echo-Planar Auslesezug (Abkürzung EPI, abgeleitet von engl. "echo-planar imaging"), mit dem man ein komplettes Bild um jedes Spin-Echo ausliest. Dabei startet man den Echozug in der Regel derart, dass die Akquisition der k-Raum-Zentrumszeile mit dem Spin-Echo zusammenfällt. Wegen des unvermeidlichen T2*-Zerfalls während des Echozugs kann in der EPI-Technik der k-Raum nur linear kodiert werden, d.h., bei einem symmetrischen Auslesen des k-Raums wird die k-Raum-Mitte in der Mitte des Echozugs ausgelesen. Die Dauer eines Echozugs beträgt ca. 64 ms (128 akquirierte Zeilen, effektiver Echoabstand 0.5 ms). D.h., von der halben Echozeit TE/2 zwischen HF-Refokussierungspuls und Spin-Echo wird die Auslesezeit $TA_1 \sim 32ms$ zum Auslesen der frühen Echos benötigt (siehe Figur 1) und steht damit nicht zur Diffusionspräparation zur Verfügung. Während der gleich langen halben Echozeit TE/2 zwischen HF-Anregungspuls und HF-Refokussierungspuls steht aber nur die Zeit, während der die HF-Pulse ausgespielt werden, nicht zur Diffusionspräparation zur Verfügung. Die Dauer eines HF-Pulses beträgt in der Regel aber nur wenige Millisekunden, ist also kurz im Vergleich zur Dauer des EPI-Auslesezugs. Theoretisch wäre zwar eine asymmetrische Akquisition des k-Raums möglich, bei der man weniger k-Raum-Zeilen vor der k-Raum Zentrumszeile als nach der k-Raum Zentrumszeile akquiriert und damit die Zeit $TA_1$ in Figur 1 verkürzt, dies kann aber zum kompletten Signalverlust führen, da das tatsächliche Echo im k-Raum, in Folge von makroskopischer Bewegung während der Diffusionspräparation, häufig verschoben ist.

Die ungleichen Zeiten vor und nach dem HF-Refokussierungspuls werden erfindungsgemäß durch die Wahl einer ungeraden Anzahl von Diffusionskontrast-Gradientenpulsen erreicht, da bei einer solchen Konstellation in Kombination mit den weiteren erfindungsgemäßen Merkmalen, nämlich dass das globale nullte Gradientenmoment und das globale erste Gradientenmoment den Wert Null aufweisen, der zeitliche Mittelpunkt der Diffusionskontrast-Gradientenpulssequenz nicht mit dem Zeitpunkt des RF-Refokussierungspulses zusammenfällt. Durch die Wahl der ungeraden Zahl von Diffusionskontrast-Gradientenpulsen und der genannten Merkmale wird weiter erreicht, dass die Summe der nullten Gradientenmomente der Gradientenpulse die in dem Zeitraum zwischen Anregungs-HF-Puls und Beginn des HF-Refokussierungspuls und/oder in dem Zeitraum zwischen dem Zeitpunkt des Ende des HF-Refokussierungspulses und dem Zeitpunkt des Beginns des Auslesevorgangs nicht den Wert Null ergibt. Auf diese Weise wird erreicht, dass Zeitintervalle während der keine Diffusions-Gradientenpulse geschaltet werden können, wie beispielsweise während des HF-Refokussierungspulses, auch zur Diffusionssensibilisierung, also dem b-Wert, beitragen.

Infolge der höheren Diffusionssensibilisierung erreicht man mit dem erfindungsgemäßen Verfahren eine weitere Reduktion der Echozeit. Zusätzlich kann man bei dem erfindungsgemäßen Verfahren auf allen drei Achsen simultan Gradienten zur Minimierung der Echozeit schalten. Diese zusätzliche Maßnahme kann das erfindungsgemäße Verfahren bei dem Bestreben nach Reduktion der Echozeit ergänzen bzw. damit kombiniert werden.

Eine erfindungsgemäße Diffusionskontrast-Pulssequenz kann als Ansteuersequenz, welche auf einem Magnetresonanzbildgebungssystem zur Ansteuerung des Magnetresonanzbildgebungssystems ausführbar ist, vorliegen und einen HF-Anregungspuls, ein Auslesemodul und eine Diffusionskontrast-Gradientenpulssequenz mit genau einem HF-Refokussierungspuls umfassen. Die Diffusionskontrast-Gradientenpulssequenz weist eine ungerade Anzahl von Diffusionskontrast-Gradientenpulsen auf. Die Diffusionskontrast-Gradientenpulse sind derart dimensioniert und positioniert, dass die Summe der nullten Gradientenmomente der Diffusionskontrast-Gradientenpulse den Wert Null aufweist und die Summe der ersten Gradientenmomente der Diffusionskontrast-Gradientenpulse den Wert Null aufweist. Unter einem Auslesemodul soll in diesem Zusammenhang eine Teilsequenz verstanden werden, die Gradientenpulse unter anderem zur Frequenzkodierung und Phasenkodierung der empfangenen Signale sowie ein oder mehrere Zeitfenster umfasst, in dem ein Messsignal in Form eines Echosignals erfasst werden kann. Des Weiteren kann das Auslesemodul weitere HF-Pulse und/oder Gradientenpulse zur wiederholten Formation von mehreren Echosignale sowie deren Kodierung enthalten.

Mit der erfindungsgemäßen Diffusionskontrast-Gradientenpulssequenz werden wie bei der herkömmlichen geschwindigkeitskompensierten Sequenz die Artefakte infolge makroskopischer Bewegung wie der Herzbewegung reduziert. Die minimale Echozeit, die benötigt wird, um eine gewünschte Diffusions-sensitivität zu erreichen, ist bei der erfindungsgemäßen Diffusionskontrast-Gradientenpulssequenz in der Regel aber signifikant kürzer. Somit ist das Signal/Rausch-Verhältnis der berechneten Bildern höher und damit die Messzeitverlängerung gegenüber einer Stjeskal-Tanner-Sequenz (siehe Figur 1) geringer, als es bei der in Figur 2 gezeigten, herkömmlichen geschwindigkeitskompensierten Pulssequenz der Fall ist. Die geringere Messzeitverlängerung ergibt sich auch daraus, dass aufgrund des verbesserten Signal/Rausch-Verhältnisses Messungen zur Durchschnittsbildung nicht so oft wiederholt werden müssen.

Die erfindungsgemäße Pulssequenz ist geschwindigkeitskompensiert und ist damit, wie die geschwindigkeitskompensierte Sequenz im Stand der Technik, weniger empfindlich gegenüber makroskopischer Bewegung als die nicht geschwindigkeitskompensierte Stejskal-Tanner-Sequenz. In der abdominellen diffusionsgewichteten Bildgebung werden dadurch insbesondere Artefakte infolge der Herzbewegung, die bei Messungen mit der Stejskal-Tanner-Sequenz (siehe Figur 1) immer wieder berichtet werden, vermieden. Insbesondere ist die minimale Echozeit, die benötigt wird, um eine gewünschte Diffusionssensitivität zu erreichen, bei der erfindungsgemäßen Ansteuersequenz immer dann kürzer als bei einer herkömmlichen geschwindigkeitskompensierten Sequenz, wie sie in Figur 2 gezeigt ist, wenn unterschiedlich lange Zeitintervalle zur Diffusions-Sensibilisierung vor und nach dem HF-Refokussierungspuls zur Verfügung stehen. In diesem Fall kann mit der erfindungsgemäßen Ansteuersequenz eine gewünschte Diffusionssensitivität mit einer kürzeren Echozeit realisiert werden, als es bei der herkömmlichen, in Figur 2 gezeigten geschwindigkeitskompensierten Sequenz möglich ist. Wie bereits erwähnt, hat die kürzere Echozeit zur Folge, dass das Signal/Rausch-Verhältnis der berechneten Bilder höher und damit die Messzeitverlängerung gegenüber einer Stejskal-Tanner-Sequenz (siehe Figur 1) geringer ist. Bei Geweben mit kurzer transversaler Relaxationszeit (z.B. Leber-Parenchym, T2-40 ms bei 1.5T) begrenzt der T2-Zerfall auch die maximale Echozeit, bei der Bildgebung noch sinnvoll ist. Bei vorgegebener Echozeit ist die maximale Diffusions-Sensibilisierung, die in der gegebenen Zeit erreicht werden kann, unter den genannten Voraussetzungen mit der erfindungsgemäßen Sequenz höher als mit der geschwindigkeitskompensierten Sequenz (in Figur 2 gezeigt) im Stand der Technik.

Ein Ansteuersequenz-Ermittlungssystem ist dazu ausgebildet, eine beschriebene Ansteuersequenz zu ermitteln, welche auf einem Magnetresonanzbildgebungssystem ausführbar ist. Beispielsweise wird von dem Ansteuersequenz-Ermittlungssystem auf der Basis von Parameterdaten, die zum Beispiel aus einem Messprotokoll stammen, eine geeignete Pulssequenz bzw. Ansteuersequenz ermittelt.

Das erfindungsgemäße Magnetresonanzbildgebungssystem umfasst eine Steuereinrichtung, welche zur Steuerung des Magnetresonanzbildgebungssystems unter Nutzung des erfindungsgemäßen Verfahrens ausgebildet ist. Dabei kann das erfindungsgemäße Magnetresonanzbildgebungssystem ein oben beschriebenes Ansteuersequenz-Ermittlungssystem umfassen.

Die wesentlichen Komponenten der beschriebenen Ansteuersequenz-Ermittlungseinrichtung und der erfindungsgemäßen Steuereinrichtung können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen zwischen einzelnen Funktionskomponenten, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete Steuereinrichtungen oder Ansteuersequenz-Ermittlungseinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Steuereinrichtung eines Magnetresonanzbildgebungssystems ladbar ist, mit Programmabschnitten, um alle Schritte des erfindungsgemäßen Verfahrens auszuführen, wenn das Programm in der Steuereinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen

Zum Transport zur Steuereinrichtung oder der Ansteuersequenz-Ermittlungseinrichtung und/oder zur Speicherung an oder in der Steuereinrichtung oder der Ansteuersequenz-Ermittlungseinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Steuereinrichtung oder der Ansteuersequenz-Ermittlungseinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Eine weitgehend softwaremäßige Realisierung hat den Vorteil, dass auch schon bisher verwendete Steuereinrichtungen oder Ansteuersequenz-Ermittlungseinrichtungen auf einfache Weise durch ein Software-Update nachgerüstet werden können, um auf die erfindungsgemäße Weise zu arbeiten. Insofern wird die Aufgabe auch durch ein Computerprogramm gelöst, welches direkt in eine Speichereinrichtung eines Magnetresonanzsystems ladbar ist, mit Programmabschnitten, um alle Schritte des erfindungsgemäßen Verfahrens auszuführen, wenn das Programm in dem Magnetresonanzsystem ausgeführt wird.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung auch die verschiedenen Merkmale unterschiedlicher Ausführungsbeispiele und Ansprüche auch zu neuen Ausführungsbeispielen kombiniert werden, sofern dadurch der durch die Ansprüche definierte Schutzumfang der Erfindung nicht verlassen wird.

In einer Ausgestaltung des erfindungsgemäßen Verfahrens sind von der ungeraden Anzahl, d.h. $2n+1$ Diffusionskontrast-Gradientenpulsen, die ersten bis n-ten Diffusionskontrast-Gradientenpulse zu den $n+2$-ten bis $2n+1$-ten Diffusionskontrast-Gradientenpulsen jeweils paarweise punktsymmetrisch bezüglich des zeitlichen Mittelpunkts der Diffusionskontrast-Gradientenpulssequenz und der $n+1$-te Diffusionskontrast-Gradientenpuls ist achsensymmetrisch zu einer in Ordinatenrichtung verlaufenden Symmetrieachse durch den zeitlichen Mittelpunkt der Diffusionskontrast-Gradientenpulssequenz und weist ein nulltes Gradientenmoment auf, das betragsmäßig doppelt so groß wie die Summe der nullten Gradientenmomente der 1-ten bis n-ten Gradientenpulse ist. Eine solche konkrete Anordnung und Dimensionierung der Diffusionskontrast-Gradientenpulse gewährleistet, dass die Summe der nullten und ersten Gradientenmomente der Diffusionskontrast-Gradientenpulse den Wert Null aufweist und somit eine geschwindigkeitskompensierte Pulssequenz erzielt wird. Weiterhin ist gewährleistet, dass die Summe der zeitlich dem an der Symmetrieachse der Diffusionskontrast-Gradientenpulssequenz positionierten Diffusionskontrast-Gradientenpuls vorausgehenden Diffusionskontrast-Gradientenpulse nicht Null ist, so dass zumindest ein Teil der Zeiten zwischen den Diffusionskontrast-Gradientenpulsen zur Diffusionssensibilisierung genutzt werden kann, da in diesen Zeiten die angeregten Spins dephasiert sind und somit auch die Zeitspanne zwischen Gradienten, während der keine Gradienten anliegen, zum b-Wert beiträgt.

In einer bevorzugten Variante des erfindungsgemäßen Verfahrens liegt der zeitliche Mittelpunkt der Diffusionskontrast-Gradientenpulssequenz zeitlich vor dem Zeitpunkt des HF-Refokussierungspulses. Wie bereits erwähnt, ist die minimale Echozeit, die benötigt wird, um eine gewünschte Diffusions-sensitivität zu erreichen, bei dem erfindungsgemäßen Verfahren immer dann kürzer als bei einer herkömmlichen geschwindigkeitskompensierten Sequenz, wie sie in Figur 2 gezeigt ist, wenn unterschiedlich lange Zeitintervalle zur Diffusions-Sensibilisierung vor und nach dem HF-Refokussierungspuls zur Verfügung stehen. Dies wird in der bevorzugten Variante des erfindungsgemäßen Verfahrens durch Verschiebung des zeitlichen Mittelpunkts der Diffusionskontrast-Gradientenpulssequenz vor den Zeitpunkt des HF-Refokussierungspulses erreicht. Im diesem Fall sind im Regelfall die Zeitintervalle zur Diffusions-Sensibilisierung vor dem HF-Refokussierungspuls länger als nach dem HF-Refokussierungspuls.

Besonders vorteilhaft ist es, wenn bei dem erfindungsgemäßen Verfahren die Summe der nullten Gradientenmomente von zwei direkt aufeinander folgenden Diffusionskontrast-Gradientenpulsen einen von Null verschiedenen Wert aufweist. Wie bereits erwähnt, ist dieses Merkmal Voraussetzung dafür, dass auch Zeitintervalle ohne Gradientenschaltung die unmittelbar auf die zwei zeitlich benachbarten Diffusionskontrast-Gradientenpulsen folgen zur Diffusionssensibilisierung beitragen.

In einer besonders effektiven Variante des erfindungsgemäßen Verfahrens ist der HF-Refokussierungspuls zwischen den n+1-ten und den n+2-ten Diffusionskontrast-Gradientenpuls geschaltet. Dabei weist der n+1-te Diffusionskontrast-Gradientenpuls bevorzugt das gleiche Vorzeichen auf wie der n-te Diffusionskontrast-Gradientenpuls.

In einer alternativen Ausgestaltung des erfindungsgemäßen Verfahrens ist der HF-Refokussierungspuls zwischen den n-ten und den n+1-ten Diffusionskontrast-Gradientenpuls geschaltet und weist der n+1-te Diffusionskontrast-Gradientenpuls das umgekehrte Vorzeichen auf wie der n-te Diffusionskontrast-Gradientenpuls.

In einer sehr praktikablen Variante des erfindungsgemäßen Verfahrens weisen alle Diffusionskontrast-Gradientenpulse betragsmäßig die gleiche Amplitude auf und die Dauer der einzelnen Diffusionskontrast-Gradientenpulse ist derart gewählt, dass die komplette zur Diffusionssensibilisierung zur Verfügung stehende Zeit genutzt wird. In diesem Fall wird ein optimales Ergebnis erreicht, was die Minimierung der erforderlichen Echozeit der verwendeten Pulssequenz betrifft. Unter einer Nutzung der kompletten zur Diffusionssensibilisierung zur Verfügung stehenden Zeit soll verstanden werden, dass die Echozeit mit Ausnahme der Zeitintervalle, in denen der HF-Anregungspuls und das Auslesemodule geschaltet werden, für die Diffusionssensibilisierung verwendet wird.

Bevorzugt weist das Auslesemodul einen EPI-Echozug auf. Mit EPI-Echozügen können alle k-Raum-Zeilen nach einer HF-Anregung ausgelesen werden.

In einer besonders effektiven und leicht zu realisierenden Ausgestaltung des erfindungsgemäßen Verfahrens ist n = 2, so dass 5 Gradientenpulse geschaltet werden. Bei dieser Anzahl von Gradientenpulsen kann die Echozeit besonders kurz gehalten werden. Dies liegt daran, dass bei einer möglichst kleinen Anzahl von Gradientenpulsen die Summe der Rampenzeiten für das Hochfahren von Gradienten besonders kurz ist.

In einer besonders bevorzugten Variante des erfindungsgemäßen Verfahrens, insbesondere, wenn n = 2 ist, sind das Vorzeichen des ersten und des zweiten Diffusionskontrast-Gradienten-pulses unterschiedlich.

In einer alternativen Ausgestaltung des erfindungsgemäßen Verfahrens mit einer Anzahl von fünf Diffusions-Gradientenpulsen beträgt die zeitliche Dauer des zweiten und des vierten Diffusionskontrast-Gradientenpulses den Wert Null. In diesem Fall beträgt die Anzahl der Diffusionskontrast-Gradientenpulse tatsächlich nur drei. Diese spezielle Variante ist genau dann besonders effizient, wenn die Zeit die vor dem, HF-Refokussierungspuls zur Diffusionssensibilisierung zur Verfügung steht sehr viel länger ist als die Zeit die nach dem HF-Refokussierungspuls zur Diffusionssensibilisierung zur Verfügung steht.

In einer schichtselektiven Variante des erfindungsgemäßen Verfahrens wird bei dem Anregungsvorgang synchron mit dem HF-Anregungspuls mindestens ein Schichtselektionsgradient in Schichtselektionsrichtung erzeugt. Weiterhin wird bevorzugt bei dem Auslesevorgang synchron mit dem HF-Refokussierungspuls ein Schichtselektionsgradient erzeugt. Mit Hilfe von Schichtselektionsgradienten lassen sich ausgewählte Schichten gezielt anregen und deren Diffusionsverhalten mit Hilfe des erfindungsgemäßen Verfahrens bildlich wiedergeben.

Die Erfindung wird im Folgenden unter Hinweis auf die beigefügten Figuren anhand von Ausführungsbeispielen noch einmal näher erläutert. Es zeigen:

Figur 1 ein Sequenzdiagramm, welches eine Stejskal-Tanner-Pulssequenz darstellt,

Figur 2 ein Sequenzdiagramm, in dem eine herkömmliche flusskompensierte Diffusionskontrast-Pulssequenz dargestellt wird,

Figur 3 ein Flussdiagramm, welches ein Verfahren gemäß einem Ausführungsbeispiel der Erfindung veranschaulicht,

Figur 4 ein Sequenzdiagramm, in dem eine Diffusionskontrast-Pulssequenz gemäß einem ersten Ausführungsbeispiel der Erfindung veranschaulicht wird,

Figur 5 ein Sequenzdiagramm, in dem eine Diffusionskontrast-Pulssequenz gemäß einem zweiten Ausführungsbeispiel der Erfindung veranschaulicht wird,

Figur 6 ein Sequenzdiagramm, in dem eine Diffusionskontrast-Pulssequenz gemäß einem dritten Ausführungsbeispiel der Erfindung veranschaulicht wird,

Figur 7 ein Magnetresonanzbildgebungssystem gemäß einem Ausführungsbeispiel der Erfindung.

In Figur 1 ist eine Stejskal-Tanner-Sequenz gezeigt. Dies ist die mit Abstand wichtigste diffusionsgewichte Pulssequenz im Stand der Technik. In der ersten Zeile des Schaubilds, welche mit RD/ADC gekennzeichnet ist, sind ein HF-Anregungspuls 1, welcher zu Beginn einer Pulssequenz gleichzeitig mit einem Schichtselektionsgradienten 6 (siehe zweite Zeile GS) geschaltet wird, und ein HF-Refokussierungspuls 2 gezeigt, der zwischen zwei Diffusionskontrast-Gradientenpulsen 4, 5 (siehe dritte Zeile GDW) geschaltet wird und mit dem zeitgleich ebenfalls ein Schichtselektionsgradient 7 (siehe zweite Zeile GS) geschaltet wird. Die Gradientenpulse 4, 5 haben gleiche Polarität und in der Regel gleiche Amplitude und Dauer. Der HF-Refokussierungspuls 2 formiert ein Spinecho 3 (siehe erste Zeile), das im gezeichneten Beispiel mit einem EPI-Echoauslesezug EPIR, umfassend eine Mehrzahl von Auslesefenstern, ausgelesen wird. Weiterhin ist in dem Schaubild in Figur 1 in der zweituntersten Zeile ein Gradientenschema GR in Ausleserichtung (Frequenzkodierrichtung) und in der untersten Zeile ein Gradientenschema GP in Phasenkodierrichtung gezeigt.

Die Echozeit TE ist die Zeit zwischen dem HF-Anregungspuls und dem Echo 3. Sie lässt sich über den zeitlichen Abstand TE/2 zwischen HF-Anregungs- und HF-Refokussierungspuls justieren, der halb so lang ist wie die Echozeit TE. Für das in Figur 1 gezeigte Stejskal-Tanner-Schema mit symmetrischen trapezförmigen Diffusions-Gradienten 4, 5 mit der Amplitude G erhält man für den b-Wert:

$$b_{stejskal} = 4\pi^2 \ \gamma^2 G^2 [\delta^2 (\Delta - \delta/3) + T_r^3/30 - \delta RT^2/6]. \qquad (10)$$

Dabei ist $T_r$ die Rampenzeit (engl. "ramp time"), also die Dauer einer der beiden Flanken des Gradienten, und $\delta$ die sogenannte Dauer eines trapezförmigen Gradienten, die als Summe aus der Dachzeit (engl. "Flat top time") und einer Rampenzeit des Gradienten definiert ist. Die Dachzeit ist dabei das Zeitintervall, während dessen die Amplitude des Gradienten konstant ist. $\Delta$ ist die Zeit, die zwischen dem Einschalten der beiden Gradientenpulse 4, 5 vergeht. Die eben definierten Zeitintervalle sind in Figur 1 visualisiert. In Formel (10) wird nur der Beitrag der Diffusionskontrast-Gradienten 4 und 5 zum b-Wert der Sequenz berücksichtigt. Die starken Diffusionskontrast-Gradienten 4,5 der Stejskal-Tanner-Sequenz machen die Sequenz also nicht nur empfindlich auf ungerichtete molekulare Brownsche Bewegung, sondern ungewünscht auch extrem empfindlich gegenüber makroskopischer Bewegung, da das Signal von bewegten Spins dephasiert wird. Diese Dephasierung lässt sich mit Gradienten-Schemata vermeiden (bzw. signifikant verringern), deren erstes Moment zu einem gewünschten Zeitpunkt Null ist. Werden nach diesem Zeitpunkt keine Gradienten mehr in die betrachtete Richtung geschaltet, ist das erste Moment nach Formel (1) auch für alle späteren Zeiten Null. Man spricht in diesem Zusammenhang von Geschwindigkeits- bzw. flusskompensierten Gradienten-schemata. Allgemein wird die Technik, bestimmte Momente einer Gradienten-Anordnung derart zu konzipieren, dass sie den Wert Null annehmen, auch als "Gradient Moment Nulling" bezeichnet.

Ein solches Diffusion-Gradientenschema, dessen erstes Moment gleich Null ist und das damit insensitiv gegenüber makroskopischer Bewegung ist, ist in Figur 2 gezeigt. Figur 2 zeigt eine herkömmliche flusskompensierte Diffusionskontrast-Sequenz. Sie besteht aus jeweils zwei trapezförmigen Diffusionskontrast-Gradienten 9, 10, 11, 12, die zeitlich vor und zeitlich nach dem HF-Refokussierungspuls 2 geschaltet werden. Das erste Gradientenmoment $m_1$ der Anordnung hat am Ende des vierten Gradienten 12 den Wert Null. Bei der Berechnung des Gradientenmoments ist zu beachten, dass der HF-Refokussierungspuls 2 die Phase negiert, die unmittelbar vor seinem Isodelaypunkt (bei symmetrischen Pulsen der zeitliche Mittelpunkt des HF-Refokussierpulses) akkumuliert wurde. Bei der Berechnung eines Gradientenmoments mit Hilfe der Formel (1) für eine Zeit t nach dem HF-Refokussierungspuls 2 ist also das Vorzeichen solcher Gradienten 9, 10, die vor dem

HF-Refokussierungspuls 2 geschaltet werden, umzudrehen. Unter Beachtung dieser Vorzeichenregel ist die Gradienten-Anordnung GDW symmetrisch zu ihrem zeitlichen Mittelpunkt (der in Figur 2 mit dem Mittelpunkt des HF-Refokussierungspulses 2 zusammenfällt). Für symmetrische Gradienten-Anordnungen gilt:

$$m_1(t) = m_0(t - t_{sym}) \, . \qquad (11)$$

Dabei ist $t_{sym}$ der Zeitpunkt, zu dem die Gradientenanordnung symmetrisch ist. Das nullte Moment $m_0$ der vier Gradienten 9, 10, 11, 12 akkumuliert sich zu Null. Damit folgt aus Formel (11) unmittelbar, dass das erste Moment der Anordnung zu allen Zeiten nach Ende des letzten Gradienten 12 Null ist. Alternativ kann man dieses Ergebnis natürlich auch durch direkte Integration mit Hilfe der Formel (1) berechnen.

Für den b-Wert der Gradienten-Anordnung in Figur 2 erhält man mit Hilfe der Formeln (5),(6):

$$b_{vc} = 4\pi^2 \ \gamma^2 G^2 [\,(4/3)\delta^3 + 2\delta^2 T_r + T_r^3/15 - \delta \ T_r^2/3\,] \, . \qquad (12)$$

Im Folgenden wird die Diffusionssensitivität der beiden Pulssequenz-Schemata aus den Figuren 1 und 2 verglichen. Die Echozeit TE, die Dauer der HF-Pulse $TRF_1$, $TRF_2$, sowie die Dauer $TA_1$ der Zeit zwischen Beginn des EPI-Auslesemoduls EPIR und der Akquisition der k-Raum-Zeile mit dem kleinsten Phasenkodiermoment seien vorgegeben. Um zu einem einfach vergleichbaren Resultat zu kommen, wird angenommen, dass die Rampenzeit $T_r$ vernachlässigbar ist, so dass $T_r = 0$ gesetzt werden kann. Ferner ist $T_{Diff2} = TE/2 - TA1 - TRF_2$ die zur Diffusions-Sensibilisierung nach dem HF-Refokussierungspuls zur Verfügung stehende Zeit.

Für das Stejskal-Tanner-Schema aus Figur 1 erhält man mit der Rampenzeit $T_r = 0$, $\delta = T_{Diff2}$ aus Formel (10) :

$$b_{Stejskal} = 4\pi^2 \ \gamma^2 G^2 \delta^2 (\Delta - \delta/3) = 4\pi^2 \ \gamma^2 G^2 T_{Diff}^2 (\Delta - T_{Diff2}/3) \, . \qquad (13)$$

Da der zeitliche Abstand $\Delta$ der beiden Diffusionskontrast-Gradienten 4, 5 immer größer ist als die Dauer eines Diffusionskontrast-Gradienten ($\Delta > \delta$) ist, gilt weiter:

$$b_{Stejskal} > 4\pi^2 \ \gamma^2 G^2 T_{Diff2}^2 (2 T_{Diff}/3) = (8/3)\pi^2 \ \gamma^2 G^2 T_{Diff2}^3 \, . \qquad (14)$$

Für das geschwindigkeitskompensierte Schema aus Figur 2 erhält man mit $T_r = 0$, $\delta = T_{Diff2}/2$:

$$b_{vc} = 4\pi^2 \ \gamma^2 G^2 (4/3) \delta^3 = (16/3) \ \pi^2 \ \gamma^2 G^2 \ \delta^3 = 2/3 \ \pi^2 \ \gamma^2 G^2 \ T_{Diff2}^3 \, . \qquad (15)$$

Die gewünschte Insensitivität gegenüber makroskopischer Bewegung des Schemas aus Figur 2 geht also mit einem Verlust an Diffusionssensitivität von mehr als einem Faktor 4 einher. In der Praxis ist häufig eine gewünschte Diffusions-Sensitivität $b_0$ vorgegeben. Für die zum Erreichen dieser Diffusions-Sensitivität benötigte Zeit zur Diffusions-Sensibilisierung $T_{Diff2,fc}$ erhält man durch Umformen der Formeln (14), (15):

$$T_{Diff2,fc} = \sqrt[3]{\frac{3}{2} \frac{1}{\pi^2 \gamma^2 G^2}} = \sqrt[3]{4 \frac{3}{8} \frac{1}{\pi^2 \gamma^2 G^2}} > \sqrt[3]{4} T_{Diff2,Stjeskal} \approx 1{,}6 \, T_{Diff2,Stejskal} \, . \qquad (16)$$

Dabei ist $T_{Diff2,Stejskal}$ die Zeit zur Diffusions-Sensibilisierung nach dem HF-Refokussierungspuls, die mit dem Stejskal-Tanner-Schema zum Erreichen der gleichen Diffusions-Sensitivität $b_0$ benötigt wird.

Die Zeit zur Diffusions-Sensibilisierung und damit die für den T2-Zerfall relevante Echozeit verlängern sich also um einen Faktor 1,6. Wegen der relativ kurzen T2-Relaxationszeit von Leberparenchym ist diese längere Echozeit bei der Kontrastbildgebung im Bereich der Leber mit einem drastischen Signalverlust verbunden, der nur durch signifikant längere Akquisitionszeiten kompensiert werden kann.

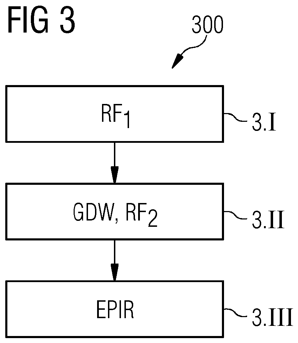

In Figur 3 ist ein Flussdiagramm gezeigt, mit dem ein Verfahren 300 zur Ansteuerung eines Magnetresonanzbildgebungssystems zur Erzeugung von Magnetresonanzbilddaten BD eines Untersuchungsobjekts P gemäß einem Ausführungsbeispiel der Erfindung, bei dem Magnetresonanzrohdaten RD erfasst werden, veranschaulicht wird.

Das Verfahren umfasst einen Anregungsvorgang, wobei bei dem Schritt 3.I ein HF-Anregungspuls $RF_1$ erzeugt wird. Zeitlich nachfolgend wird bei dem Schritt 3.II eine Diffusionskontrast-Gradientenpulssequenz GDW erzeugt. Die Diffusionskontrast-Gradientenpulssequenz GDW umfasst erfindungsgemäß eine ungerade Anzahl von 2n+1 zeitlich sequentiell geschalteten Diffusionskontrast-Gradientenpulsen 13, 14, 15, 16, 17, wobei die Summe der nullten Gradientenmomente $m_0$ der Diffusionskontrast-Gradientenpulse 13, 14, 15, 16, 17 den Wert Null aufweist und die die Summe

der ersten Gradientenmomente $m_1$ der Diffusionskontrast-Gradientenpulse 13, 14, 15, 16, 17 den Wert Null aufweist. Außerdem umfasst die Diffusionskontrast-Gradientenpulssequenz einen HF-Refokussierungspuls 2, der zwischen zwei der Diffusionskontrast-Gradientenpulse eingestrahlt wird. Der HF-Refokussierungspuls hat den Zweck, dass Spins, deren Signal, beispielsweise in Folge von lokalen Off-Resonanzen, zwischen Anregungs-und HF-Refokussierungspuls dephasiert wurde, nach dem HF-Refokussierungspuls rephasiert werden. Zu einem Echozeitpunkt TE der außerhalb der Dauer der GDW liegt, ist das Signal vollständig rephasiert.

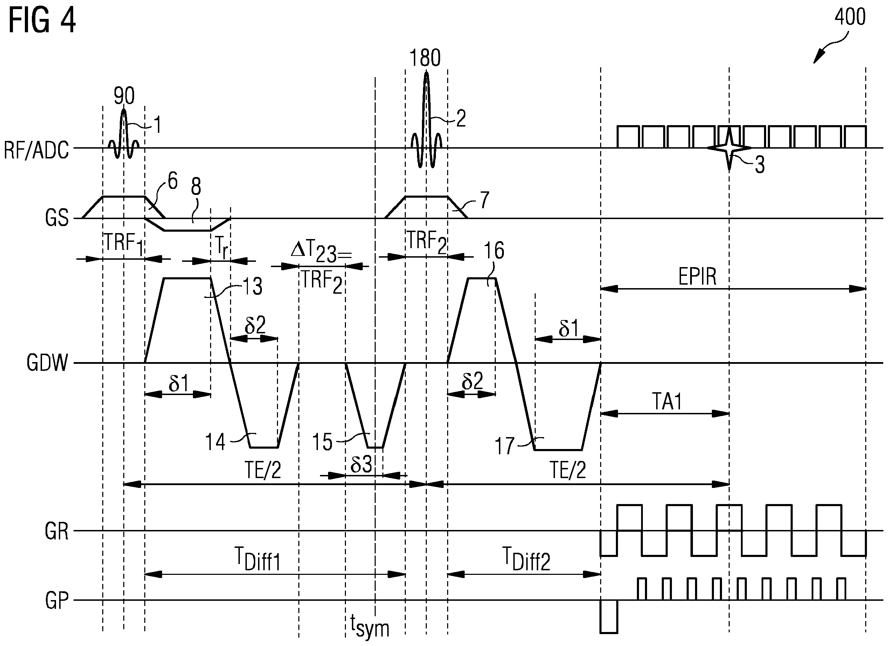

Weiterhin weist das Verfahren 300 bei dem Schritt 3.III einen Auslesevorgang auf, bei dem in einem oder mehreren Zeitfenstern Magnetresonanzrohdaten erfasst werden. Das Ausleseintervall EPIR umfasst diese Zeitfenster. Der Echozeitpunkt liegt innerhalb des Ausleseintervalls. Genauer gesagt, startet das der Akquisition von Magnetresonanzrohdaten zugeordnete Ausleseintervall EPIR bereits schon um eine Zeit TA1 vor dem Echozeitpunkt TE und erstreckt sich zeitlich auch um etwa die Zeit $TA_1$ über den Zeitpunkt der Echozeit TE hinaus (siehe Figur 4).

In Figur 4 ist eine Diffusionskontrast-Pulssequenz 400 gemäß einem ersten Ausführungsbeispiel der Erfindung gezeigt. Die Diffusionswichtung erfolgt mit einer Sequenz GDW von fünf Gradientenpulsen 13, 14, 15, 16, 17. Die Zeitintervalle $\Delta T_{23}$ zwischen Ende des zweiten Gradienten 14 und Beginn des dritten Gradienten 15 sowie $TRF_2$ zwischen Ende des dritten Gradienten 15 und Beginn des vierten Gradienten 16 sind gleich lang. In dem zweiten Zeitintervall $TRF_2$ wird in der in Figur 4 dargestellten Ausführungsform ein HF-Refokussierungspuls 2 geschaltet. Die zeitliche Mitte des dritten Gradienten 15 fällt mit der Symmetrieachse 18 der Diffusionskontrast-Gradientenpulssequenz GDW zusammen. Der dritte Gradient 15 ist spiegelsymmetrisch zur Symmetrieachse 18. Der erste 13 und fünfte Gradient 17 sowie der zweite 14 und der vierte Gradient 16 sind jeweils punktsymmetrisch zum Symmetriezentrum 19 (zeitlicher Mittelpunkt der GradientenPulssequenz GDW).

Unter Beachtung der im Zusammenhang mit Formel (11) genannten Vorzeichenregel für Spin-Echo-Sequenzen ist die Gradienten-Anordnung also symmetrisch. Somit beträgt der Wert des ersten Moments $m_1$ für alle Zeiten nach dem Ende des fünften Gradienten 17 Null, sofern das nullte Moment $m_0$ der Gradientenpulsfolge GDW verschwindet. Letzteres ist eine notwendige Voraussetzung, um überhaupt ein Echo 3 zum Zeitpunkt TE nach dem HF-Anregungspuls 1 zu formieren. Das nullte Moment $m_0$ ist genau dann Null, wenn das nullte Moment des dritten Gradienten 15 betragsmäßig doppelt so groß ist wie der Betrag der vorzeichenbehafteten Summe der nullten Momente von erstem 13 und zweiten 14 bzw. vierten 16 und fünften Gradient 17.

Werden symmetrische Trapezgradienten mit gleicher Rampenzeit $T_r$ und gleichem Betrag der Amplitude G eingesetzt, gilt also:

$$\delta_3 = 2(\delta_1-\delta_2). \qquad (17)$$

Für den b-Wert der Gradienten-Pulsfolge GDW in Figur 4 erhält man aus den Gleichungen 3,4 und nach Elimination von $\delta_3$ mit Hilfe der Formel (17):

$$b_{vc5} = 4\pi^2\gamma^2G^2 \ [(4/3)\delta_1^3 + 3\delta_1^2T_r + \delta_2^2T_r - 2\delta_1\delta_2T_r - (1/3)\delta_1T_r^2 + (1/20)T_r^3 + 2(\delta_1-\delta_2)^2T_{RF2}]. \qquad (18)$$

Um zu einem anschaulichen Vergleich mit dem geschwindigkeitskompensierten Schema aus Figur 2 zu gelangen, wird wiederum angenommen, dass die Rampenzeit $T_r = 0$ vernachlässigbar kurz ist, und es werden zwei Spezialfälle betrachtet:

Im ersten Spezialfall sei angenommen, dass die Zeit, die zur Diffusions-Sensibilisierung nach dem HF-Refokussierungspuls 2 zur Verfügung steht, $T_{Diff2} = TE/2-TA1-T_{RF2}/2$, gleich der Zeit $T_{Diff1}$ ist, die vor dem HF-Refokussierungspuls zur Verfügung steht (In den gezeichneten Beispielen gilt $T_{Diff1} = TE/2 - T_{RF1}/2 - T_{RF2}/2$), also $T_{Diff2} = T_{Diff1}$.

Die maximale Diffusions-Sensibilisierung in der gegebenen Zeit erhält man, wenn man $\delta_1 = \delta_2$ und damit nach Formel (17): $\delta_3 = 0$.

In diesem Grenzfall verschwindet der dritte Gradient 15 und das Schema ist identisch mit dem Schema aus Figur 2. Entsprechend erhält man durch Einsetzen von $\delta_1 = \delta_2 = T_{Diff2}/2$, $T_r = 0$:

$$b_{vc5}(1) = 4\pi^2 \ \gamma^2G^2 \ [ \ (4/3)\delta_1^3]= 4\pi^2 \ \gamma^2G^2 \ [ \ (4/3)(T_{Diff2}/2)^3] = (2/3) \ \pi^2 \ \gamma^2G^2 \ T_{Diff2}^3 = b_{vc}. \qquad (19)$$

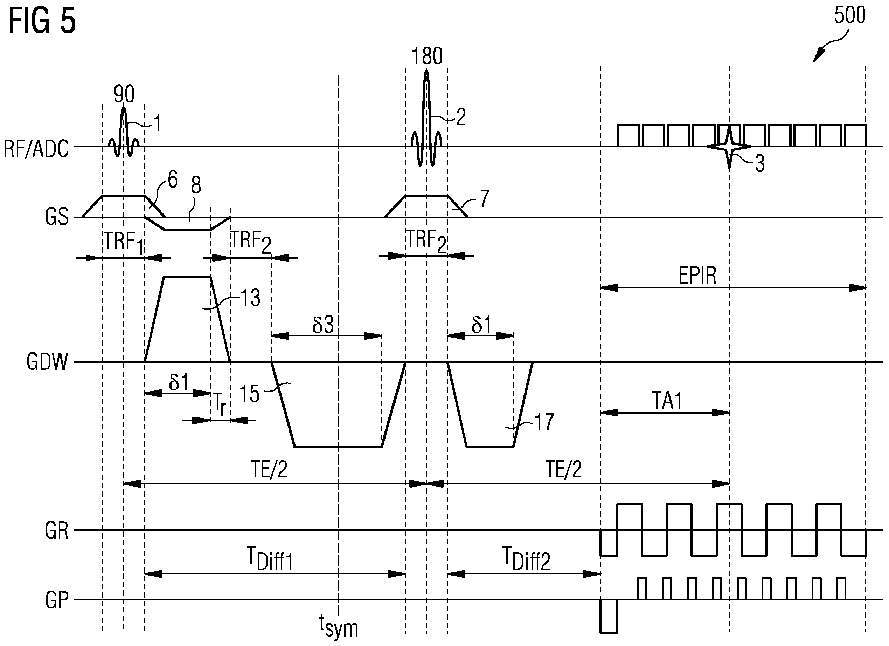

Wird angenommen, dass $T_{Diff1} \geq 3T_{Diff2} + T_{RF2}-T_r$, was der in Figur 5 gezeigten Pulssequenz entspricht, so erhält man in diesem Fall mit $\delta_2 = 0$ und $\delta_1 = T_{Diff2}-T_r$ die maximale Diffusionswichtung. Der zweite 14 und vierte Gradient 16 verschwinden also und der dritte Gradient 15 ist nach Formel (16) doppelt so lang wie der erste Gradient 13 bzw.

der fünfte Gradient 17, also $\delta_3 = 2\delta_1$. Durch Einsetzen in Formel (18) erhält man mit $\delta_1 = T_{Diff2}$:

$$b_{vc5}(2) = 4\pi^2 \ \gamma^2 G^2 \ [ \ (4/3)\delta_1^3 + 2\delta_1^2 T_{RF2} ]$$
$$= 4\pi^2 \ \gamma^2 G^2 \ [ \ (4/3) \ T_{Diff2}^3 + 2T_{Diff2}^2 T_{RF2} ]. \tag{20}$$

Vernachlässigt man den zweiten Term, also die Diffusions-Sensibilisierung während der ersten Pause und während des Schaltens des HF-Refokussierungspulses 2, so erhält man:

$$b_{vc5}(2) > (16/3) \ \pi^2 \ \gamma^2 G^2 \ T_{Diff2}^3 = 8 \ (2/3) \ \pi^2 \ \gamma^2 G^2 \ T_{Diff2}^3 = 8 \ b_{vc}. \tag{21}$$

In diesem Grenzfall ist die Diffusions-Sensibilisierung des erfindungsgemäßen Schemas also mehr als einen Faktor 8 höher als die Diffusions-Sensibilisierung bei Verwendung des in Figur 2 gezeigten herkömmlichen geschwindigkeitskompensierten Schemas. Die zum Erreichen einer gewünschten Diffusions-Sensitivität $b_0$ benötigte Zeit zur Diffusions-Sensibilisierung $T_{Diff2,fc5}$ ist entsprechend um einen Faktor 2 reduziert:

$$T_{Diff2,fc5} = \sqrt[3]{\frac{1}{8}} T_{Diff2,fc} = \frac{1}{2} T_{Diff2,fc}. \tag{22}$$

Die für den T2-Zerfall relevante Echozeit kann also im Vergleich zu der in Figur 2 gezeigten Pulssequenz halbiert werden.

Um zu einem Vergleich mit dem Stejskal-Tanner-Schema zu gelangen, führt man auch hier die Zeit $\Delta$ zwischen dem ersten Gradient 13 und dem fünften Gradient 17 ein. Diese beträgt im betrachteten Grenzfall

$$\Delta = 3\delta_1 + 2T_{RF2} = 3T_{Diff2} + 2 \ T_{RF2}. \tag{23}$$

Auflösen von Gleichung (23) nach $T_{RF2}$ und einsetzen in Gleichung (20) liefert:

$$b_{vc5}(2) = 4\pi^2 \ \gamma^2 G^2 \ [ \ (4/3) \ T_{Diff2}^3 + 2T_{Diff2}^2 T_{RF2} ] = 4\pi^2 \ \gamma^2 G^2 \ T_{Diff2}^2$$
$$[\Delta - (5/3) T_{Diff2}] < 4\pi^2 \ \gamma^2 G^2 T_{Diff}^2 (\Delta - T_{Diff2}/3) = b_{Stejskal}. \tag{24}$$

Die Diffusions-Sensibilisierung ist also auch bei dem erfindungsgemäßen geschwindigkeitskompensierten Schema echt kleiner als die des nicht geschwindigkeitskompensierten Stejskal-Tanner-Schemas, wobei jedoch die erfindungsgemäße Pulssequenz vollständig geschwindigkeitskompensiert ist.

Der folgende Abschnitt befasst sich mit der optimalen Auslegung der erfindungsgemäßen Pulssequenz. Es wird angenommen, dass die Zeit $T_{Diff1}$, die vor dem HF-Refokussierungspuls 2 und die Zeit $T_{Diff2}$, die nach dem HF-Refokussierungspuls 2 zur Diffusions-Sensibilisierung zur Verfügung steht, vorgeben ist. Ferner sei eine maximale Gradientenamplitude $G_{max}$ sowie eine maximale Gradientenanstiegsrate $S_{max}$ (engl. "gradient slew rate") vorgegeben. Aufgabe ist es, die Dauer der fünf Gradientenpulse derart festzulegen, dass eine maximale Diffusions-Sensibilisierung (also ein maximaler b-Wert) in der gegeben Zeit erreicht wird. Dabei können die Zeiten $T_{Diff1}$ und $T_{Diff2}$ auch implizit über eine gewünschte Echozeit TE, die Dauer der RF-Pulse $TRF_1$ und $TRF_2$ und die Zeitspanne $TA_1$ von Beginn des Auslesemoduls EPIR bis zum Echozeitpunkt TE sowie die Dauer weiterer zu schaltender Module vorgegeben sein.

Zunächst berechnet man die kürzeste Rampenzeit $T_r$, in der man unter Beachtung der vorgegeben maximalen Gradientenanstiegszeit $S_{max}$ einen Gradienten bis zur maximalen Gradientenamplitude $G_{max}$ rampen kann. Diese Zeit wählt man als gemeinsame Rampenzeit aller Gradienten. Als nächstes legt man die Dauer der fünf Gradienten fest. Die optimale Dauer der Gradienten hängt dabei von der relativen Länge der Zeitintervalle $T_{Diff1}$ und $T_{Diff2}$ ab:

Sind die Zeit $T_{Diff1}$, die vor dem HF-Refokussierungspuls 2 zur Diffusions-Sensibilisierung zur Verfügung steht und die Zeit $T_{Diff2}$, die nach dem HF-Refokussierungspuls zur Diffusions-Sensibilisierung zur Verfügung steht, gleich lang, so gelangt man zu dem bereits besprochenen Grenzfall, bei dem die erfindungsgemäße Pulssequenz in die herkömmliche, in Figur 2 gezeigte Pulssequenz übergeht.

Für den Fall, dass die Zeiten $T_{Diff1}$, $T_{Diff2}$, $T_{RF2}$ und $T_r$ so gewählt sind, dass gilt $T_{Diff1} \geq 3T_{Diff2} + T_{RF2} - T_r$, so gelangt man, wie bereits erwähnt, zu einer Pulsfolge gemäß einem zweiten Ausführungsbeispiel der Erfindung mit der in Figur 5 gezeigten Gestalt.

In diesem Grenzfall, bei dem die vor dem HF-Refokussierungspuls zur Diffusions-Sensibilisierung zur Verfügung stehende Zeit $T_{Diff1}$ sehr viel länger ist als die nach dem HF-Refokussierungspuls zur Diffusions-Sensibilisierung zur Verfügung stehende Zeit $T_{Diff2}$, erhält man die maximale Diffusions-Sensibilisierung mit folgenden Gradientendauern:

$$\delta_1 = \delta_5 = T_{Diff2} - T_r$$

$$\delta_2 = \delta_4 = 0$$

$$\delta_3 = 2\delta_1 = 2(T_{Diff2} - T_r)$$

$$\Delta T_{23} = T_{RF2}. \tag{25}$$

Mit dieser Wahl reduziert sich das Schema mit fünf Gradienten 13, 14, 15, 16, 17 aus der Figur 4 auf das in Figur 5 gezeigte Schema 500 mit drei Gradienten 13, 15, 17, da der zweite Gradient 14 und der vierte Gradient 16 die Dauer Null haben. Anders als bei der Pulssequenz in Figur 4 wird in dem hier betrachteten Grenzfall ($T_{Diff1} \geq 3T_{Diff2} + T_{RF2} - T_r$) die komplette nach dem HF-Refokussierungspuls zur Verfügung stehende Zeit $T_{Diff2}$ zur Diffusions-Sensibilisierung genutzt.

Von der vor dem HF-Refokussierungspuls zur Verfügung stehenden Zeit $T_{Diff1}$ wird ein Zeitintervall

$$T_{Diff1,eff} = \delta1 + \delta_3 + 2T_r + T_{RF2} = 3(T_{Diff2} - T_r) + 2T_r + T_{RF2} = 3T_{Diff2} - T_r + T_{RF2} \leq T_{Diff1} \tag{26}$$

zur Diffusions-Sensibilisierung genutzt.

Setzt man dagegen $T_{Diff2} < T_{Diff1} < 3T_{Diff2} + T_{RF2} - T_r$, so gelangt man zu dem praktisch weitaus wichtigsten Fall, der dem ersten Ausführungsbeispiel entspricht, welches in Figur 4 gezeigt ist. Für eine optimale Diffusionssensibilisierung sollten bei dieser Ausführungsform folgende Bedingungen erfüllt sein:

i) $\delta_3 = 2(\delta_1 - \delta_2)$ (Formel (17)) →

i') $2\delta_1 - 2\delta_2 - \delta_3 = 0$

ii) $\delta_1 + \delta_2 + \delta_3 + 3T_r + T_{RF2} = T_{Diff1}$ (komplette Nutzung der Zeit $T_{Diff1}$)

iii) $\delta_1 + \delta_2 + 2T_r = T_{Diff2}$ (komplette Nutzung der Zeit $T_{Diff2}$). $\tag{27}$

Die Lösung des Gleichungssystems mit drei Unbekannten ergibt:

$$\delta_1 = \delta_5 = (T_{Diff1} + T_{Diff2} - 5T_r - T_{RF2})/4$$

$$\delta_2 = \delta_4 = (3T_{Diff2} - T_{Diff1} + 3T_r + T_{RF2})/4$$

$$\delta_3 = (T_{Diff2} - T_{Diff1}) - 4T_r - T_{RF2}$$

$$\Delta T_{23} = T_{RF2}. \tag{28}$$

In dem optimalen Grenzfall mit den gemäß Gleichung (28) gesetzten Parametern wird also die Dauer der einzelnen Diffusionskontrast-Gradientenpulse 13, 14, 15, 16, 17 derart gewählt, dass die komplette zur Diffusionssensibilisierung zur Verfügung stehende Zeit $T_{Diff1} + T_{Diff2}$ genutzt wird.

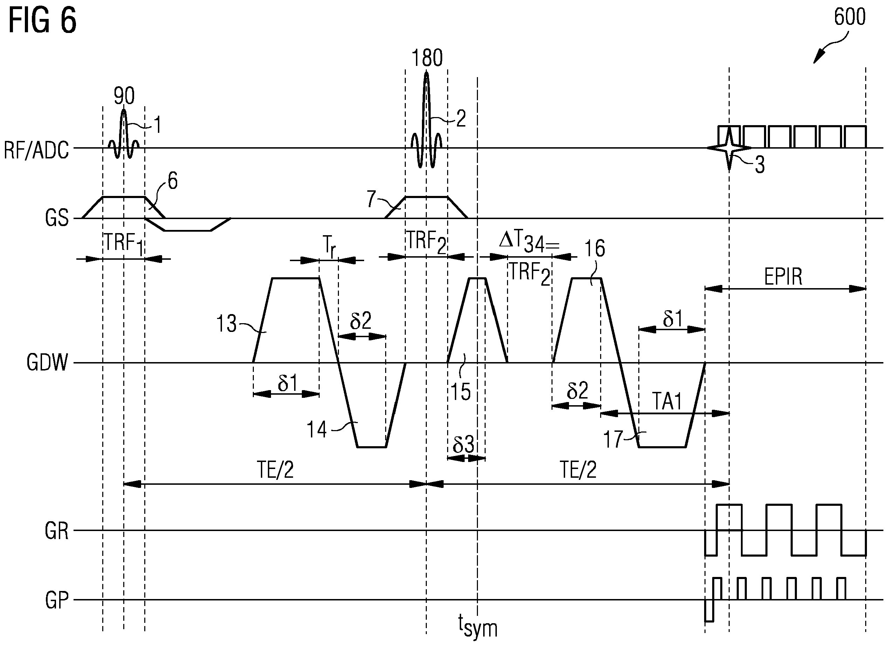

In Figur 6 ist ein Schaubild gezeigt, in dem eine Pulssequenz 600 gemäß einem dritten Ausführungsbeispiel der Erfindung veranschaulicht wird. Bei diesem Ausführungsbeispiel wird $T_{Diff2} > T_{Diff1}$ gesetzt.

Steht nach dem HF-Refokussierungspuls 2 mehr Zeit zur Diffusions-Sensibilisierung zur Verfügung als vor dem HF-Refokussierungspuls, so werden alle Diffusionskontrast-Gradienten 13, 14, 15, 16, 17 auf der Zeitachse derart verschoben, dass der HF-Refokussierungspuls 2 in die Lücke zwischen dem zweiten Gradienten 14 und dem dritten Gradienten 15 fällt. Es entsteht nun eine neue gleich lange Lücke zwischen dem dritten Gradienten 15 und dem vierten Gradienten 16, während der keine Gradienten geschaltet werden. Das relative Vorzeichen des dritten Gradienten 15 im Vergleich zu den ersten und zweiten Gradienten 13, 14 muss man umdrehen, da dieser jetzt nach dem HF-Refokussierungspuls 2 geschaltet wird. Praktisch relevant könnte der Fall $T_{Diff1} > T_{Diff2}$ werden, wenn ein sehr langer HF-Anregungspuls 1 verwendet wird, z.B. für eine zwei- oder dreidimensionale lokalisierte Anregung (Siemens-Produktname ZOOMIt) oder unmittelbar nach dem HF-Anregungspuls 1 noch ein zusätzlicher Echozug zum Beispiel zur Spulenkalibrierung akquiriert wird (siehe DE 102009014461/US 8,570,034 B2 und DE 102009014498/US 8,461,840 B2).

Bei den Grenzfallbetrachtungen wurde angenommen, dass die Zeiten $T_{Diff1}$ und $T_{Diff2}$, die zur Diffusions-Sensibilisierung zur Verfügung stehen, z.B. implizit über eine gewünschte Echozeit TE, die Dauer der HF-Pulse 1, 2 $TRF_1$ und $TRF_2$ und die Zeitspanne $TA_1$ vorgegeben sind und die Dauer der Gradientenpulse 13, 14, 15, 16, 17 gesucht wird, mit der man in der gegebenen Zeit die maximale Diffusions-Sensibilisierung erreicht. Praktisch relevanter ist der Fall, dass eine gewünschte Diffusions-Sensibilisierung (als b-Wert) vorgegeben ist und die kürzestmögliche Echozeit TE gesucht ist, mit der man diesen b-Wert realisieren kann. Dieses Problem kann aber auf das eben gelöste Problem zurückgeführt werden, indem man eine Echozeit vorgibt, für diese die Zeiten $T_{Diff1}$ und $T_{Diff2}$ berechnet und daraus die maximale Diffusions-Sensibilisierung mit den Formeln (22)-(24) sowie (17) ermittelt. Ist dieser b-Wert zu klein, erhöht man die vorgegebene Echozeit TE schrittweise so lange, bis der gewünschte b-Wert gerade realisiert werden kann. Ist der zuerst berechnete b-Wert dagegen größer als der vorgegebene b-Wert, dekrementiert man die vorgegebene Echozeit TE entsprechend. Statt der erschöpfenden Suche kann man natürlich die optimale Echozeit z.B. auch mit einem schnellen Bisektionsverfahren effektiv suchen.

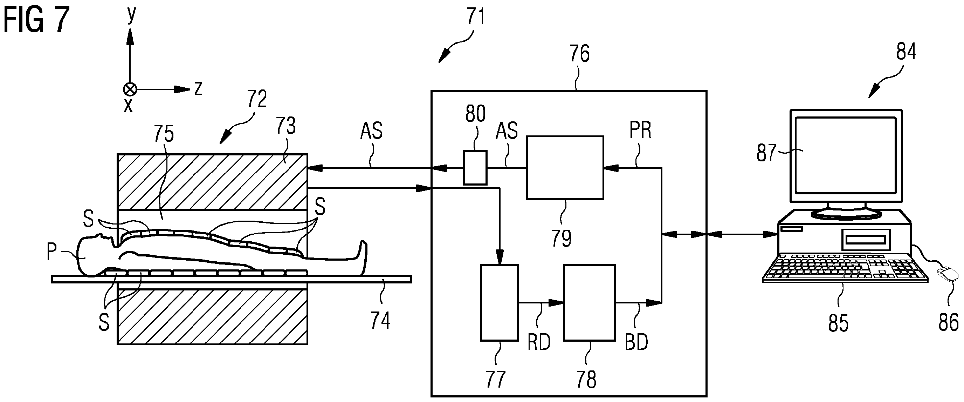

Figur 7 zeigt ein Ausführungsbeispiel einer erfindungsgemäßen Magnetresonanzanlage 71, welche in der Lage ist, nach dem erfindungsgemäßen Verfahren zu arbeiten. Kernstück dieser Magnetresonanzanlage 71 ist der Magnetresonanztomograph 72 selbst, in welchem ein Patient P auf einem Patientenlagerungstisch 74 (auch Patientenliege 74 genannt) in einem ringförmigen Grundfeldmagneten 73, welcher den Messraum 75 umschließt, positioniert wird. Auf und ggf. auch unter dem Patienten befindet sich beispielsweise eine Vielzahl von Lokalspulen S, auch Magnetresonanzspulen genannt.

Die Patientenliege 74 ist in Längsrichtung, d. h. entlang der Längsachse des Tomographen 72, verschiebbar. Diese Richtung wird in dem ebenfalls dargestellten Raumkoordinatensystem als z-Richtung bezeichnet. Innerhalb des Grundfeldmagneten befindet sich im Tomographen 72 eine nicht näher dargestellte Ganzkörperspule, mit der Hochfrequenzpulse ausgesendet und empfangen werden können. Außerdem weist der Tomograph 72 in üblicher, in der Figur nicht dargestellter Weise Gradientenspulen auf, um in jeder der Raumrichtungen x, y, z einen Magnetfeldgradienten anlegen zu können.

Angesteuert wird der Tomograph 72 von einer Steuereinrichtung 76, welche hier separat dargestellt ist. An die Steuereinrichtung 76 ist ein Terminal 84 angeschlossen. Dieses Terminal 84 weist einen Bildschirm 87, eine Tastatur 85 und ein Zeigegerät 86 für eine graphische Benutzeroberfläche, beispielsweise eine Maus 86 oder dergleichen auf. Das Terminal 84 dient u. a. als Benutzerschnittstelle, über die ein Bediener die Steuereinrichtung 76 und damit den Tomographen 72 bedient. Sowohl die Steuereinrichtung 76 als auch das Terminal 84 können auch integraler Bestandteil des Tomographen 72 sein.

Das Magnetresonanzsystem 71 kann darüber hinaus auch alle weiteren üblichen Komponenten bzw. Merkmale solcher Systeme aufweisen, wie z. B. Schnittstellen zum Anschluss eines Kommunikationsnetzes, beispielsweise eines Bildinformationssystems o. Ä. Alle diese Komponenten sind jedoch der besseren Übersichtlichkeit wegen in der Figur 7 nicht dargestellt.

Über das Terminal 84 kann ein Bediener mit der Steuereinrichtung 76 kommunizieren und so für die Durchführung der gewünschten Messungen sorgen, indem beispielsweise der Tomograph 72 von der Steuereinrichtung 76 so angesteuert wird, dass die erforderlichen Hochfrequenzpulssequenzen durch die Hochfrequenz-Spulen ausgesendet werden und die Gradientenspulen in geeigneter Weise geschaltet werden. Über die Steuereinrichtung 76 werden auch die vom Tomographen kommenden, für die Bildgebung benötigten Rohdaten RD akquiriert. Hierzu weist die Steuereinrichtung 76 eine Rohdatenerfassungseinheit 77 auf, in der von dem Tomographen 72 kommende Messsignale in Rohdaten RD gewandelt werden. Beispielsweise wird dies durch eine Demodulierung und anschließende Digitalisierung der Messsignale erreicht. In einer Signalauswerteeinheit 78, bei der es sich z. B. um ein Modul der Steuereinrichtung 76 handeln kann, werden Rohdaten RD zu Bilddaten BD rekonstruiert. Die Bilddaten BD können beispielsweise auf dem Bildschirm 87 des Terminals 84 visualisiert werden und/oder in einem Speicher hinterlegt bzw. über ein Netzwerk versandt werden. Zur Ausführung des erfindungsgemäßen Verfahrens weist die Steuereinrichtung 76 eine Ansteuersequenz-Ermittlungseinheit 79 auf, mit der eine Ansteuersequenz AS ermittelt wird, welche zum Beispiel die Pulssequenz 400 umfasst. Beispielsweise empfängt die Ansteuersequenz-Ermittlungseinheit 79 von dem Terminal 84 Protokolldaten PR, welche vorbestimmte Parameterwerte einer zu ermittelnden Pulssequenz 400 aufweisen. Weiterhin umfasst die Steuereinrichtung 76 auch eine Ansteuersequenz-Erzeugungseinheit 80, welche dazu eingerichtet ist, eine Ansteuersequenz AS, umfassend die erfindungsgemäße Pulssequenz 400, auf dem Magnetresonanztomographen 72 auszuspielen, so dass das erfindungsgemäße Verfahren 300 zur Ansteuerung eines Magnetresonanzbildgebungssystems zur Erzeugung von Magnetresonanzbilddaten BD eines Untersuchungsobjekts P ausgeführt wird.

Die zur Umsetzung der Erfindung in einem Magnetresonanzsystem 71 erforderlichen Komponenten wie die Rohdatenerfassungseinheit 77, die Signalauswerteeinheit 78, die Ansteuersequenz-Ermittlungseinheit 79 oder die Ansteuersequenz-Erzeugungseinheit 80 können zumindest teilweise oder auch vollständig in Form von Softwarekomponenten erstellt werden. Übliche Magnetresonanzsysteme weisen ohnehin programmierbare Steuereinrichtungen auf, so dass auf diese Weise die Erfindung bevorzugt mit Hilfe von geeigneter Steuersoftware realisierbar ist. D. h. es wird ein entsprechendes Computerprogramm direkt in den Speicher einer programmierbaren Steuereinrichtung 76 des betreffenden Magnetresonanzsystems 71 geladen, welches Programmcode-Mittel aufweist, um das erfindungsgemäße Verfahren 300 durchzuführen. Auf diese Weise sind auch bereits existierende Magnetresonanzsysteme einfach und kostengünstig nachrüstbar.

Insbesondere ist es möglich, dass einige der Komponenten auch als Unterroutinen in bereits in der Steuereinrichtung 76 vorhandenen Komponenten realisiert sind, bzw. dass vorhandene Komponenten für den erfindungsgemäßen Zweck mitverwendet werden. Dies betrifft beispielsweise die Ansteuersequenz-Ermittlungseinheit 79, die zum Beispiel in einer in einer existierenden Steuereinrichtung 76 bereits vorhandenen Ansteuersequenz-Erzeugungseinheit implementiert werden kann, welche dazu bestimmt sind, die Hochfrequenzspulen, Gradientenspulen oder sonstigen Komponenten im Tomographen in geeigneter Weise zur Durchführung einer üblichen bildgebenden Messung anzusteuern.

Erfindungsgemäß wird die Beobachtung genutzt, dass in der auf der Spin-Echo-Technik basierten Diffusions-Bildgebung vor dem HF-Refokussierungspuls 2 häufig mehr Zeit zur Diffusionspräparation zur Verfügung steht als nach dem HF-Refokussierungspuls.

Bei dem herkömmlichen, nicht flusskompensierten Stejskal-Tanner-Schema (siehe Figur 1) kann diese Zeit zur Diffusions-Sensibilisierung genutzt werden, sofern man die beiden Diffusionsgradienten nicht symmetrisch um den HF-Refokussierungspuls anordnet, sondern den ersten Gradienten-Puls 4 zeitlich so früh wie möglich schaltet, dass die Magnetisierung nach Ende des ersten Gradienten-Pulses 4 dephasiert ist und somit auch die Zeitspanne zwischen dem Ende des ersten Diffusionsgradienten 4 und dem Beginn des zweiten Diffusionsgradienten 5, während der keine Gradienten anliegen, zum b-Wert beiträgt.

Bei herkömmlichen flusskompensierten Schemata (siehe Figur 2) erhöht eine asymmetrische Anordnung der beiden Gradienten-Paare 9, 10, 11, 12 den b-Wert dagegen nicht, da die Magnetisierung nach Ende des ersten Paares 9, 10 vollständig rephasiert ist.

Bei der erfindungsgemäßen Sequenz 400 wird mit Hilfe von drei bzw. fünf Gradientenpulsen die längere Zeitspanne vor dem HF-Refokussierungspuls zur Diffusions-Sensibilisierung genutzt. Es wird abschließend noch einmal darauf hingewiesen, dass es sich bei den vorbeschriebenen Verfahren, Pulssequenzen und Vorrichtungen lediglich um bevorzugte Ausführungsbeispiele der Erfindung handelt und dass die Erfindung vom Fachmann variiert werden kann, ohne den Bereich der Erfindung zu verlassen, der durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Verfahren (300) zur Ansteuerung eines Magnetresonanzbildgebungssystems zur Erzeugung von Magnetresonanzbilddaten (BD) eines Untersuchungsobjekts (P), bei dem Magnetresonanzrohdaten (RD) erfasst werden, wobei das Verfahren mindestens eine Diffusionskontrast-Pulssequenz aufweist mit

   - einem Anregungsvorgang, wobei ein HF-Anregungspuls (1) erzeugt wird,
   - einem Auslesevorgang zur Akquisition von Magnetresonanzrohdaten (RD), welcher das Empfangen von HF-Signalen umfasst,
   - einem Schritt zur Diffusions-Sensibilisierung unter Verwendung einer Diffusionskontrast-Gradientenpulssequenz (GDW), wobei

      - die Diffusionskontrast-Gradientenpulssequenz (GDW) eine ungerade Anzahl von 2n+1 zeitlich sequentiell geschalteten Diffusionskontrast-Gradientenpulsen (13, 14, 15, 16, 17) umfasst, wobei n eine natürliche Zahl ist,
      - die Summe der nullten Gradientenmomente ($m_0$) der Diffusionskontrast-Gradientenpulse (13, 14, 15, 16, 17) den Wert Null aufweist,
      - die Summe der ersten Gradientenmomente ($m_1$) der Diffusionskontrast-Gradientenpulse (13, 14, 15, 16, 17) den Wert Null aufweist,

   **dadurch gekennzeichnet, dass** die Diffusionskontrast-Gradientenpulssequenz (GDW) genau einen HF-Refokussierungspuls (2) umfasst, wobei
   - zwischen zwei der Diffusionskontrast-Gradientenpulse (13, 14, 15, 16, 17) der eine HF-Refokussierungspuls (2) geschaltet wird.

2. Verfahren (300) nach Anspruch 1, **dadurch gekennzeichnet, dass** die ersten bis n-ten Diffusionskontrast-Gradientenpulse (13, 14) zu den n+2-ten bis 2n+1-ten Diffusionskontrast-Gradientenpulsen (16, 17) jeweils paarweise punktsymmetrisch bezüglich des zeitlichen Mittelpunkts der Diffusionskontrast-Gradientenpulssequenz (GDW) sind und der n+1-te Diffusionskontrast-Gradientenpuls (15) achsensymmetrisch zu einer in Ordinatenrichtung verlaufenden Achse durch den zeitlichen Mittelpunkt der Diffusionskontrast-Gradientenpulssequenz (GDW) ist und ein nulltes Gradientenmoment ($m_0$) aufweist, das betragsmäßig doppelt so groß wie die Summe der nullten Gradientenmomente ($m_0$) der 1-ten bis n-ten Gradientenpulse (13, 14) ist.

3. Verfahren (300) nach einem der Ansprüche 1 oder 2, **dadurch gekennzeichnet, dass** der zeitliche Mittelpunkt ($t_{sym}$) der Diffusionskontrast-Gradientenpulssequenz (GDW) zeitlich vor dem Zeitpunkt ($TRF_2$) des HF-Refokussierungspulses (2) liegt.

4. Verfahren (300) nach einem der Ansprüche 1 bis 3, **dadurch gekennzeichnet, dass** die Summe der nullten Gradientenmomente ($m_0$) von zwei direkt aufeinander folgenden Diffusionskontrast-Gradientenpulsen (13, 14, 15, 16, 17) einen von Null verschiedenen Wert aufweist.

5. Verfahren (300) nach einem der Ansprüche 1 bis 4, **dadurch gekennzeichnet, dass** der HF-Refokussierungspuls (2) zwischen den n+1-ten (15) und den n+2-ten Diffusionskontrast-Gradientenpuls (16) geschaltet wird und der n+1-te Diffusionskontrast-Gradientenpuls (15) das gleiche Vorzeichen aufweist wie der n-te Diffusionskontrast-Gradientenpuls (14).

6. Verfahren (300) nach einem der Ansprüche 1 bis 4, **dadurch gekennzeichnet, dass** der HF-Refokussierungspuls (2) zwischen den n-ten (14) und den n+1-ten Diffusionskontrast-Gradientenpuls (15) geschaltet wird und der n+1-te Diffusionskontrast-Gradientenpuls (15) das umgekehrte Vorzeichen aufweist wie der n-te Diffusionskontrast-Gradientenpuls (14).

7. Verfahren (300) nach einem der Ansprüche 1 bis 6, **dadurch gekennzeichnet, dass** alle Diffusionskontrast-Gradientenpulse (13, 14, 15, 16, 17) betragsmäßig die gleiche Amplitude aufweisen und die Dauer der einzelnen Diffusionskontrast-Gradientenpulse (13, 14, 15, 16, 17) derart gewählt ist, dass die komplette zur Diffusionssensibilisierung zur Verfügung stehende Zeit genutzt wird.

8. Verfahren (300) nach einem der Ansprüche 1 bis 7, **dadurch gekennzeichnet, dass** der Auslesevorgang ein Auslesemodul mit einem EPI-Echozug umfasst.

9. Verfahren (300) nach einem der Ansprüche 1 bis 8, **dadurch gekennzeichnet, dass** n = 2 ist.

10. Verfahren (300) nach einem der Ansprüche 1 bis 9, **dadurch gekennzeichnet, dass** das Vorzeichen des ersten und des zweiten Diffusionskontrast-Gradientenpulses (13, 14) unterschiedlich ist.

11. Verfahren (300) nach einem der Ansprüche 1 bis 10, **dadurch gekennzeichnet, dass** während dem Anregungsvorgang synchron mit dem HF-Anregungspuls mindestens ein Schichtselektionsgradient (6) in Schichtselektionsrichtung geschaltet wird und/oder bei dem Diffusions-Sensibilisierungsvorgang synchron mit dem HF-Refokussierungspuls (2) ein Schichtselektionsgradient (7) geschaltet wird.

12. Magnetresonanzbildgebungssystem (71), umfassend eine Steuereinrichtung (76), welche zur Steuerung des Magnetresonanzbildgebungssystems (71) unter Nutzung eines Verfahrens nach einem der Ansprüche 1 bis 11 ausgebildet ist.

13. Computerprogrammprodukt mit einem Computerprogramm, welches direkt in eine Speichereinrichtung einer Steuereinrichtung (76) eines Magnetresonanzbildgebungssystems (71) ladbar ist, mit Programmabschnitten, um alle Schritte des Verfahrens nach einem der Ansprüche 1 bis 11 auszuführen, wenn das Computerprogramm in der Steuereinrichtung (76) des Magnetresonanzbildgebungssystems ausgeführt wird.

14. Computerlesbares Medium, auf welchem von einer Rechnereinheit eines Magnetresonanzbildgebungssystems (71) einlesbare und ausführbare Programmabschnitte gespeichert sind, um alle Schritte des Verfahrens nach einem der Ansprüche 1 bis 11 auszuführen, wenn die Programmabschnitte von der Rechnereinheit ausgeführt werden.

**Claims**

1. Method (300) for actuating a magnetic resonance imaging system for generating magnetic resonance image data (BD) of an object under examination (P) with which magnetic resonance raw data (RD) is acquired, wherein the method has at least one diffusion contrast pulse sequence with

   - an excitation process, wherein an RF excitation pulse (1) is generated,
   - a readout process for the acquisition of magnetic resonance raw data (RD), which comprises the reception of RF signals,
   - a step for diffusion-sensitisation using a diffusion contrast gradient pulse sequence (GDW), wherein

      - the diffusion contrast gradient pulse sequence (GDW) comprises an uneven number of 2n+1 diffusion contrast gradient pulses (13, 14, 15, 16, 17) switched in chronological succession, where n is a natural number,
      - the sum of the zero gradient moments ($m_0$) of the diffusion contrast gradient pulses (13, 14, 15, 16, 17) has the value zero,
      - the sum of the first gradient moments ($m_1$) of the diffusion contrast gradient pulses (13, 14, 15, 16, 17) has the value zero,

   **characterised in that** the diffusion contrast gradient pulse sequence (GDW) comprises precisely one RF refocusing pulse (2), wherein the one RF refocusing pulse (2) is switched between two of the diffusion contrast gradient pulses (13, 14, 15, 16, 17).

2. Method (300) according to claim 1, **characterised in that** the first to n-th diffusion contrast gradient pulses (13, 14) for the n+2-th to 2n+1-th diffusion contrast gradient pulses (16, 17) are each arranged in point-symmetric pairs with respect to the temporal midpoint of the diffusion contrast gradient pulse sequence (GDW) and the n+1-th diffusion contrast gradient pulse (15) is arranged axially symmetrically to an axis extending in the ordinate direction through the temporal midpoint of the diffusion contrast gradient pulse sequence (GDW) and has a zero gradient moment ($m_0$) of twice the magnitude of the sum of the zero gradient moments ($m_0$) of the 1-th to n-th gradient pulses (13, 14).

3. Method (300) according to one of claims 1 or 2, **characterised in that** the temporal midpoint ($t_{sym}$) of the diffusion contrast gradient pulse sequence (GDW) lies chronologically before the time ($TRF_2$) of the RF refocusing pulse (2).

4. Method (300) according to one of claims 1 to 3, **characterised in that** the sum of the zero gradient moments ($m_0$) of two directly sequential diffusion contrast gradient pulses (13, 14, 15, 16, 17) has a value different from zero.

5. Method (300) according to one of claims 1 to 4, **characterised in that** the RF refocusing pulse (2) is switched between the n+1-th (15) and the n+2-th diffusion contrast gradient pulse (16) and the n+1-th diffusion contrast gradient pulse (15) has the same positive or negative sign as the n-th diffusion contrast gradient pulse (14).

6. Method (300) according to one of claims 1 to 4, **characterised in that** the RF refocusing pulse (2) is switched between the n-th (14) and the n+1-th diffusion contrast gradient pulse (15) and the n+1-th diffusion contrast gradient pulse (15) has the opposite positive or negative sign to that of the n-th diffusion contrast gradient pulse (14).

7. Method (300) according to one of claims 1 to 6, **characterised in that** all diffusion contrast gradient pulses (13, 14, 15, 16, 17) have an amplitude of the same magnitude and the duration of the individual diffusion contrast gradient pulses (13, 14, 15, 16, 17) is selected such that all time available for diffusion sensitisation is utilised.

8. Method (300) according to one of claims 1 to 7, **characterised in that** the readout process comprises a readout module with an EPI echo train.

9. Method (300) according to one of claims 1 to 8, **characterised in that** n = 2.

10. Method (300) according to one of claims 1 to 9, **characterised in that** the sign of the first and the second diffusion contrast gradient pulses (13, 14) is different.

11. Method (300) according to one of claims 1 to 10, **characterised in that**, during the excitation process, at least one slice selection gradient (6) in the slice selection direction is switched in synchronism with the RF excitation pulse and/or, during the diffusion sensitisation process, a slice selection gradient (7) is switched in synchronism with the RF refocusing pulse (2).

12. Magnetic resonance imaging system (71) comprising a control device (76) embodied to control the magnetic resonance imaging system (71) using a method according to one of claims 1 to 11.

13. Computer program product with a computer program, which can be loaded directly into a storage device of a control device (76) of a magnetic resonance imaging system (71) with program segments to execute all steps of the method according to one of claims 1 to 11 when the computer program is executed in the control device (76) of the magnetic resonance imaging system.

14. Computer-readable medium on which program segments which can be read-in and executed by a computing unit of a magnetic resonance imaging system (71) are stored in order to execute all steps of the method as claimed in one of claims 1 to 11 when the program segments are executed by the computing unit.

**Revendications**

1. Procédé (300) de commande d'un système d'imagerie par résonnance magnétique pour la production de données (BD) d'image de résonnance magnétique, d'un objet (P) à examiner, dans lequel on saisit des données (RD) brutes de résonnance magnétique, dans lequel le procédé a au moins une séquence d'impulsion de contraste de diffusion, comprenant

   - une opération d'excitation, dans laquelle on produit une impulsion (1) d'excitation HF,
   - une opération de lecture pour l'acquisition de données (RD) brutes de résonnance magnétique, qui comprend la réception de signaux HF
   - un stade de sensibilisation de diffusion en utilisant une séquence (GDW) d'impulsions de gradient de contraste de diffusion, dans lequel

      - la séquence (GDW) d'impulsions de gradient de contraste de diffusion comprend un nombre impair de $2n+1$ impulsions (13, 14, 15, 16, 17) de gradient de contraste de diffusion distribuées séquentiellement dans le temps, dans lequel n est un nombre naturel,
      - la somme des moments ($m_0$) de gradient rendus nuls des impulsions (13, 14, 15, 16, 17) de gradient de contraste de diffusion a la valeur zéro,
      - la somme des premiers moments ($m_1$) de gradient des impulsions (13, 14, 15, 16, 17) de gradient de contraste de diffusion a la valeur zéro,

      **caractérisé en ce que** la séquence (GDW) d'impulsions de gradient de contraste de diffusion comprend exactement une impulsion (2) de refocalisation HF, dans lequel

      la une impulsion (2) de refocalisation HF est mise entre deux des impulsions (13, 14, 15, 16, 17) de gradient de
      contraste de diffusion.

2. Procédé (300) suivant la revendication 1, **caractérisé en ce que** les première à $n^{ième}$ impulsions (13, 14) de gradient de contraste de diffusion sont avec les $n+2^{ième}$ à $2n+1^{ième}$ impulsions (16, 17) de gradient de contraste de diffusion symétriques respectivement par paire par rapport au point médian dans le temps de la séquence (GDW) d'impulsions de gradient de contraste de diffusion et la $n+1^{ième}$ impulsion (15) de gradient de contraste de diffusion est de symétrie par rapport à un axe s'étendant dans la direction des ordonnées et passant par le point médian dans le temps de la séquence (GDW) d'impulsions de gradient de contraste de diffusion et a un moment ($m_0$) de gradient rendu nul, qui en valeur absolue est deux fois plus grand que la somme des moments ($m_0$) de gradient rendus nuls des $1^{ième}$ à $n^{ième}$ impulsions (13, 14) de gradient.

3. Procédé (300) suivant l'une des revendications 1 ou 2, **caractérisé en ce que** le point ($t_{sym}$) médian dans le temps de la séquence (GDW) d'impulsions de gradient de contraste de diffusion est dans le temps antérieur à l'instant ($TRF_2$) de l'impulsion (2) de refocalisation HF.

4. Procédé (300) suivant l'une des revendications 1 à 3, **caractérisé en ce que** la somme des moments ($m_0$) de

gradient rendu nuls de deux impulsions (13, 14, 15, 16, 17) de gradient des contraste de diffusion se succédant directement a une valeur différente de zéro.

5. Procédé (300) suivant l'une des revendications 1 à 4, **caractérisé en ce que** l'on met l'impulsion (2) de refocalisation HF entre la n+1 $^{ième}$ impulsion (15) et la n+2$^{ième}$ impulsion (16) de gradient de contraste de diffusion et la n+1$^{ième}$ impulsion (15) de gradient de contraste de diffusion a le même signe que la n$^{ième}$ impulsion (14) de gradient de contraste de diffusion.

6. Procédé (300) suivant l'une des revendications 1 à 4, **caractérisé en ce que** l'on met l'impulsion (2) de refocalisation HF entre la n$^{ième}$ (14) et la n+1$^{ième}$ impulsion (15) de gradient de contraste de diffusion et la n+1$^{ième}$ impulsion (15) de gradient de contraste de diffusion a le signe contraire à la n$^{ième}$ impulsion (14) de gradient de contraste de diffusion.

7. Procédé (300) suivant l'une des revendications 1 à 6, **caractérisé en ce que** toutes les impulsions (13, 14, 15, 16, 17) de gradient de contraste de diffusion ont en valeur absolue la même amplitude et la durée des diverses impulsions (13, 14, 15, 16, 17) de gradient de contraste de diffusion est choisie de manière à utiliser le temps disponible complet pour la sensibilisation de diffusion.

8. Procédé (300) suivant l'une des revendications 1 à 7, **caractérisé en ce que** l'opération de lecture comprend un module de lecture ayant un train d'écho EPI.

9. Procédé (300) suivant l'une des revendications 1 à 8, **caractérisé en ce que** n = 2.

10. Procédé (300) suivant l'une des revendications 1 à 9, **caractérisé en ce que** les signes de la première et de la deuxième impulsion (13, 14) de gradient de contraste de diffusion sont différents.

11. Procédé (300) suivant l'une des revendications 1 à 10, **caractérisé en ce que**, pendant l'opération d'excitation en synchronisme avec l'impulsion d'excitation HF, on met au moins un gradient (6) de sélection de couches dans la direction de sélection de couche et/ou, lors de l'opération de sensibilisation de diffusion, on met un gradient (7) de sélection de couche en synchronisme avec l'impulsion (2) de refocalisation HF.

12. Système (71) d'imagerie par résonnance magnétique, comprenant un dispositif (76) de commande, qui est constitué pour la commande du système (71) d'imagerie par résonnance magnétique en utilisant un procédé suivant l'une des revendications 1 à 11.

13. Produit de programme d'ordinateur, comprenant un programme d'ordinateur, qui peut être chargé directement dans un dispositif de mémoire d'un dispositif (76) de commande d'un système (71) d'imagerie par résonnance magnétique, comprenant des parties de programme pour effectuer tous les stades du procédé suivant l'une des revendications 1 à 11 lorsque le programme d'ordinateur est exécuté dans le dispositif (76) de commande du système d'imagerie par résonnance magnétique.

14. Support, déchiffrable par ordinateur, sur lequel sont mises en mémoire des parties de programme pouvant être déchiffrées et exécutées par une unité informatique d'un système (71) d'imagerie par résonnance magnétique pour effectuer tous les stades du procédé suivant l'une des revendications 1 à 11 lorsque les parties de programme sont exécutés par l'unité informatique.

FIG 1

EP 3 078 979 B1

FIG 2

EP 3 078 979 B1

FIG 3

300

| RF$_1$ | 3.I |

| GDW, RF$_2$ | 3.II |

| EPIR | 3.III |

FIG 4

EP 3 078 979 B1

FIG 5

FIG 6

600

90 — 1

180 — 2

RF/ADC

3

GS

6

7

TRF$_1$

T$_r$

TRF$_2$

$\Delta T_{34} =$ TRF$_2$

16

EPIR

GDW

13

$\delta 2$

$\delta 1$

$\delta 1$

14

15

$\delta 2$

TA1

17

$\delta 3$

TE/2

TE/2

GR

GP

t$_{sym}$

EP 3 078 979 B1

FIG 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20080275329 A1
- DE 102009014461
- US 8570034 B2
- DE 102009014498
- US 8461840 B2

### In der Beschreibung aufgeführte Nicht-Patentliteratur

- **C. THOMSEN ; P. RING ; O. HENRIKSEN.** In vivo measurement of water self diffusion by magnetic resonance imaging. *Proceedings of the Seventh Scientific Meeting, Society of Magnetic Resonance in Medicine,* 1988, 890

- **MASANORI OZAKI et al.** Motion Artifact Reduction of Diffusion-Weighted MRI of the Liver: Use of Velocity-Compensated Diffusion Gradients Combined With Tetrahedral Gradients. *JOURNAL OF MAGNETIC RESONANCE IMAGING,* 2013, vol. 37, 172-178